# EUROPEAN PATENT APPLICATION

(11) **EP 4 779 313 A1**
(43) Date of publication of application: **22.07.2026**
(21) Application number: 24865505.2
(22) Date of filing: 12.09.2024
(51) Int. Cl.: G01N 33/68, C12Q 1/6869, G01N 33/50

(54) **BIOMARKER ASSOCIATED WITH AMYOTROPHIC LATERAL SCLEROSIS (ALS)**

(30) Priority: 15.09.2023 JP 2023150265
(71) Applicant: Tokushima University, Tokushima-shi, Tokushima 770-8501 (JP)
(72) Inventor: IZUMI, Yuishin, Tokushima-shi, Tokushima 770-8501 (JP); FUJITA, Koji, Tokushima-shi, Tokushima 770-8501 (JP); ITOU, Tatsuo, Fujisawa-shi, Kanagawa 251-0012 (JP); HARADA, Yuumi, Fujisawa-shi, Kanagawa 251-0012 (JP); WARUDE, Dnyaneshwar Eknath, Fujisawa-shi, Kanagawa 251-0012 (JP); MIYAKAWA, Shuuichi, Fujisawa-shi, Kanagawa 251-0012 (JP); KIKUKAWA, Yusuke, Fujisawa-shi, Kanagawa 251-0012 (JP)
(74) Representative: dompatent
(86) International application number: PCT/JP2024/032644
(87) International publication number: WO 2025/058005

(57) **Abstract**

Biomarkers for use in determining or diagnosing the progression rate or likelihood of amyotrophic lateral sclerosis (ALS), and/or in selecting or predicting therapeutic agents are provided. Biomarkers selected from the group consisting of IL-17A, KLRD1, KRT19, NCF2, TFF2, YTHDF3, Th17, regulatory T cells (Treg), mature CD8 T, naive CD8 T, exhausted CD8 T, classical monocyte, memory CD4 T, Th17/Treg, mature CD8 T/naive CD8 T, and mature CD8 T/exhausted CD8 T were found.

## Description

### TECHNICAL FIELD

The present invention relates to biomarkers associated with amyotrophic lateral sclerosis (ALS), or the use thereof, or methods of using the same.

### BACKGROUND ART

Amyotrophic lateral sclerosis (ALS) is a disease in which abnormalities occur in neurons, causing muscles of the entire body to atrophy (Non-Patent Document 1: https://www.nhk.or.jp/kenko/atc_762.html). ALS is a neurodegenerative disease in which motor neurons (nerve cells) are impaired, and is a disease in which muscle atrophy and muscle weakness progress. ALS requires artificial ventilation or results in death 2-5 years after onset. Further, ALS has a prevalence of 5-7 people per 100,000 and approximately 10,000 patients in Japan. Furthermore, it is known that ALS is approximately 90% sporadic and approximately 10% familial (such as SOD1 mutations).

For the evaluation of ALS symptoms, it is known to use the revised ALS Functional Rating Scale (ALSFRS-R). ALSFRS-R is an assessment scale created to understand the activities of daily living of ALS patients. ALSFRS-R is composed of 12 items (5 levels from 0 to 4) such as speech, swallowing, dressing and hygiene, and walking, and is evaluated by the total score (0 to 48). ALSFRS-R is used in clinical trial inclusion criteria, primary endpoints, etc.

### RELATED DOCUMENTS

### Non-Patent Documents

[Non-Patent Document 1] NHK Health Channel, "Initial symptoms and complications of ALS (amyotrophic lateral sclerosis) where muscles decline", URL: https://www.nhk.or.jp/kenko/atc_762.html

### SUMMARY OF INVENTION

### Problem to be Solved by the Invention

In ALS, the immunological profile of peripheral blood is largely unknown. In particular, the profile associated with the rate of symptom progression has not been sufficiently elucidated. If the immunological profile of peripheral blood is clarified, it is considered useful for diagnosis, patient stratification in clinical trials, surrogate markers for therapeutic intervention, etc. Therefore, there is a need for biomarkers that determine the progression rate or likelihood of ALS.

### Means for Solving the Problem

The present inventors have conducted extensive research to solve the above problems, and have found biomarkers associated with amyotrophic lateral sclerosis (ALS) or their use (for example, use for determining or diagnosing the progression rate or likelihood of amyotrophic lateral sclerosis (ALS) in a subject) or methods of using the same (for example, methods for determining or diagnosing the progression rate or likelihood of amyotrophic lateral sclerosis (ALS) in a subject).

The present inventors have also found biomarkers for use in determining or diagnosing the progression rate or likelihood of amyotrophic lateral sclerosis (ALS), and/or in selecting or predicting therapeutic agents.

The present inventors have also found a method for determining or diagnosing the progression rate or likelihood of amyotrophic lateral sclerosis (ALS) in a subject, comprising determining the level of a biomarker in a sample derived from said subject.

That is, the present invention provides the following:
(1) A biomarker for use in determining or diagnosing the progression rate or likelihood of amyotrophic lateral sclerosis (ALS), and/or in selecting or predicting a therapeutic agent, wherein the biomarker is selected from the group consisting of IL-17A, KLRD1, KRT19, NCF2, TFF2, YTHDF3, Th17, regulatory T cells (Treg), mature CD8 T, naive CD8 T, exhausted CD8 T, classical monocyte, memory CD4 T, Th17/Treg, mature CD8 T/naive CD8 T, and mature CD8 T/exhausted CD8 T.
(2) The biomarker according to (1), for use in determining or diagnosing rapidly progressive amyotrophic lateral sclerosis (ALS).
(3) The biomarker according to (1), wherein the biomarker is selected from combinations consisting of IL-17A and Th17, IL-17A and memory CD4 T, KLRD1 and mature CD8 T, TFF2 and mature CD8 T, and NCF2 and classical monocyte.
(4) A method for determining or diagnosing the progression rate or likelihood of amyotrophic lateral sclerosis (ALS) in a subject, comprising determining the level of the biomarker according to any one of (1) to (3) in a sample derived from said subject.
(5) The method according to (4), further comprising determining or diagnosing the progression rate or likelihood of ALS based on a reference level of said biomarker.
(6) The method according to (5), wherein the reference level is the level of said biomarker in a sample derived from a subject without ALS, a sample derived from a subject with ALS, a sample derived from a subject with non-rapidly progressive ALS, or a sample derived from a subject with rapidly progressive ALS.

### Effects of the Invention

According to the present invention, biomarkers associated with amyotrophic lateral sclerosis (ALS) or their use or methods of using the same are provided. According to the present invention, biomarkers for use in determining or diagnosing the progression rate or likelihood of amyotrophic lateral sclerosis (ALS), and/or in selecting or predicting therapeutic agents are provided. According to the present invention, a method for determining or diagnosing the progression rate or likelihood of amyotrophic lateral sclerosis (ALS) in a subject, comprising determining the level of a biomarker in a sample derived from said subject, is provided.

### BRIEF DESCRIPTION OF DRAWINGS

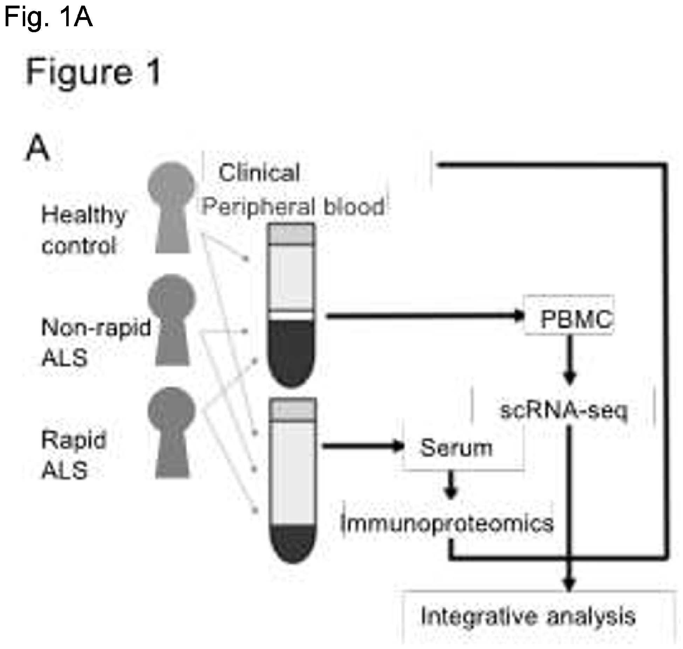

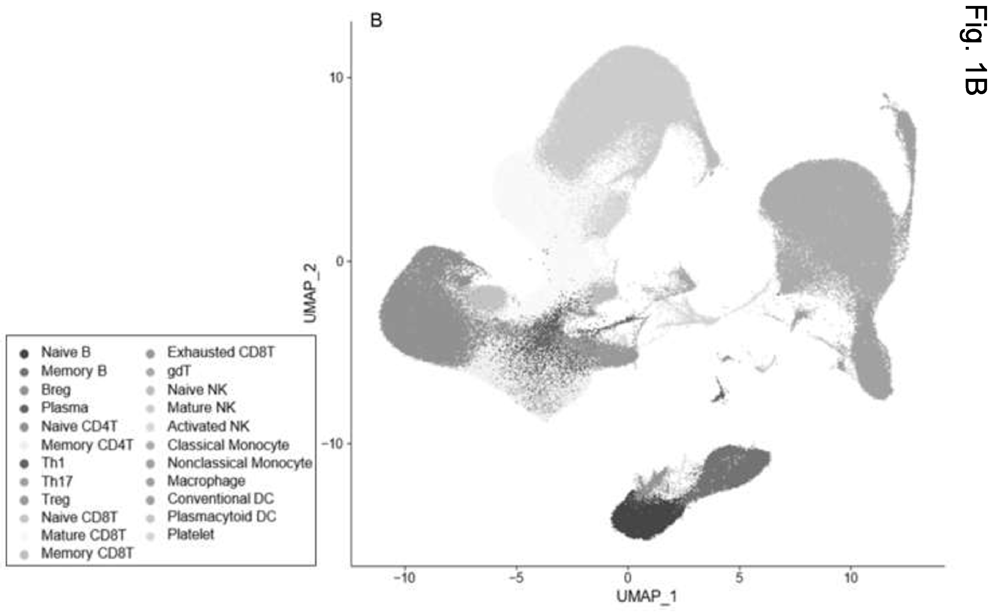

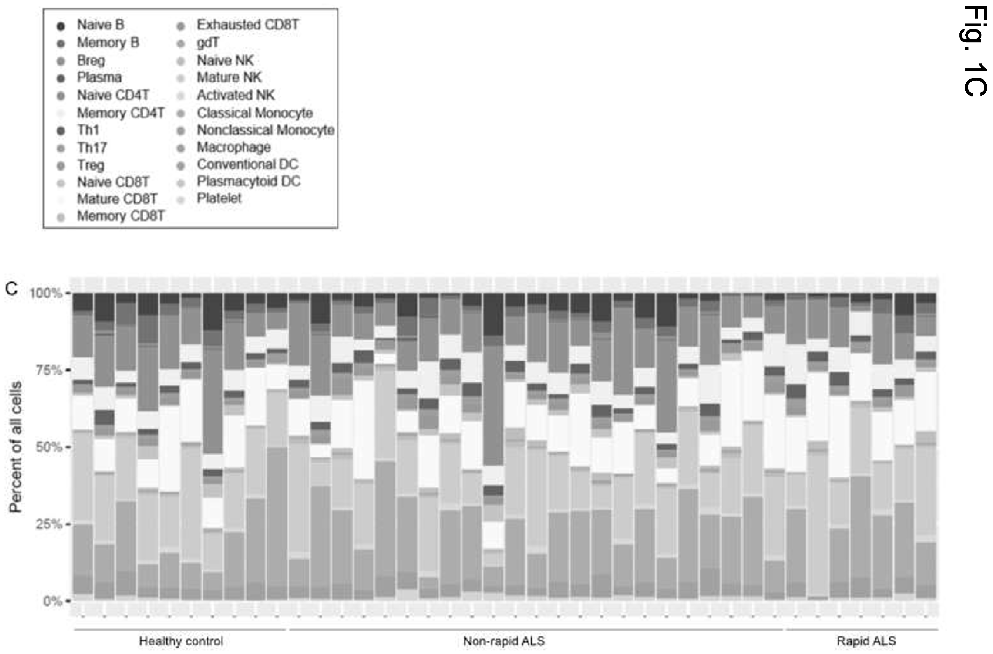

[Figure 1] Study design and cell type profiling in single-cell RNA sequencing. A) Overview of this study integrating single-cell RNA sequencing (scRNA-seq) analysis of peripheral blood mononuclear cells (PBMC), serum immunoproteomics, and clinical information for healthy controls, non-rapid amyotrophic lateral sclerosis (ALS) patients, and rapid ALS patients. B) Uniform Manifold Approximation and Projection (UMAP) showing 23 identified cell types. C) Frequency of cell types for each sample. Frequencies are displayed as the percentage of each cell type when the total number of cells in each sample is set to 100%. DC = dendritic cell, NK = natural killer.

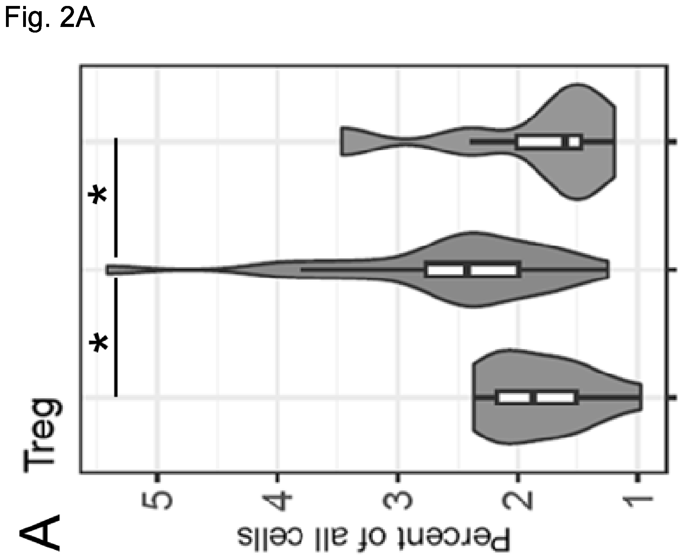

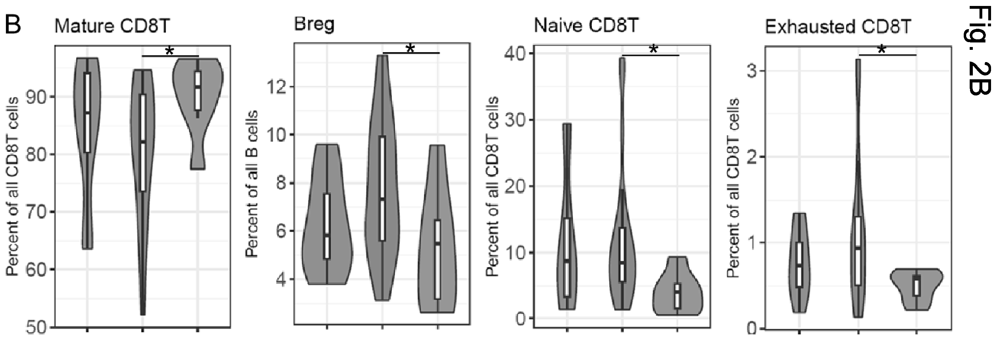

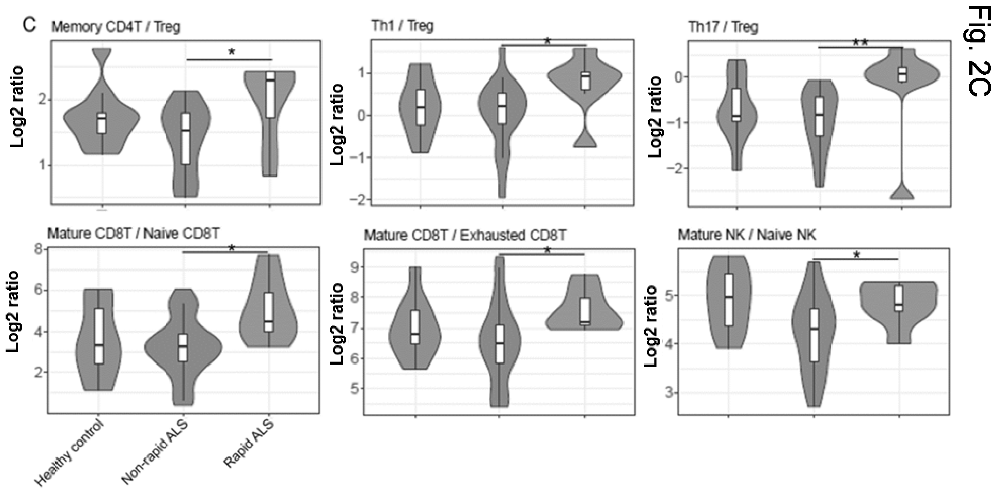

[Figure 2] Inter-group comparison of frequencies of each cell type. A) Frequency of regulatory T cells in all cells. B) Frequency of cell types in specific cell groups. C) Ratio of cell types compared to related cell types. They showed significant differences in rapid-amyotrophic lateral sclerosis (ALS) compared to non-rapid ALS by Tukey HSD test. Numbers indicate the median for each group. Lines and asterisks indicate significant combinations by Tukey HSD test (*p < 0.05, **p < 0.005, and ***p < 0.0005). Breg = regulatory B cells; NK = natural killer; Th1 = helper T1 cells; Th17 = helper T17 cells; Treg = regulatory T cells.

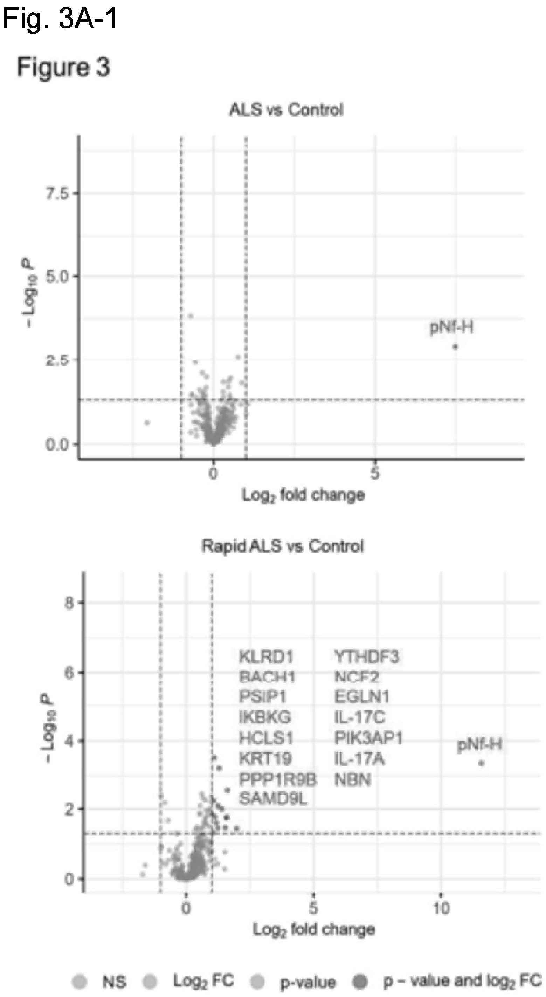

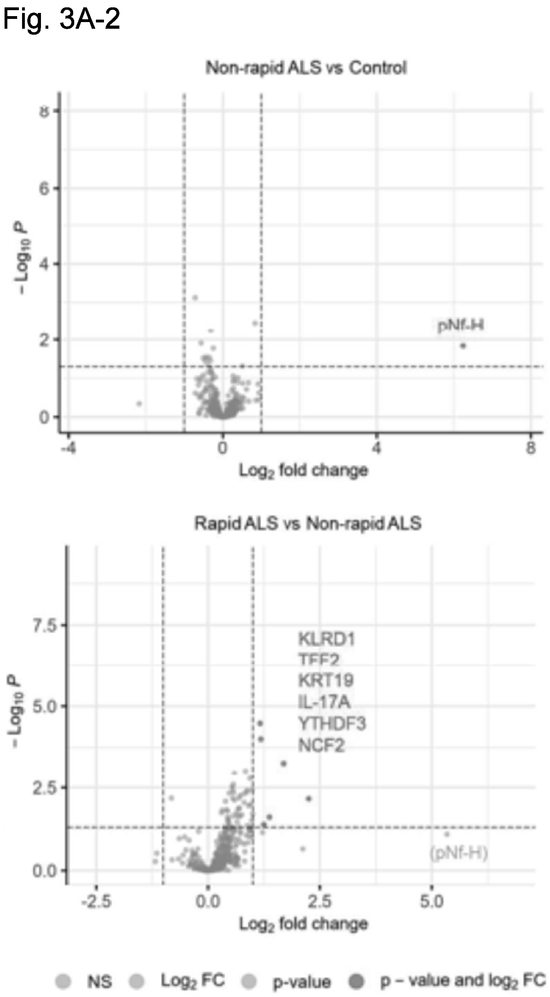

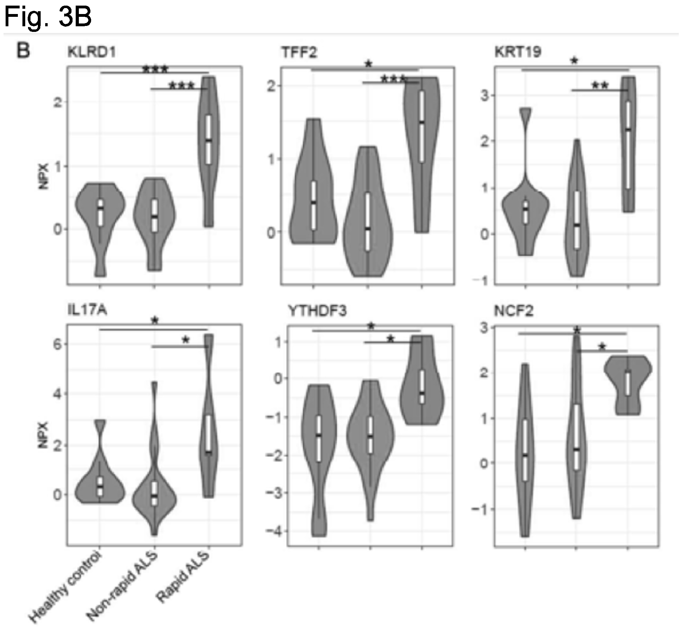

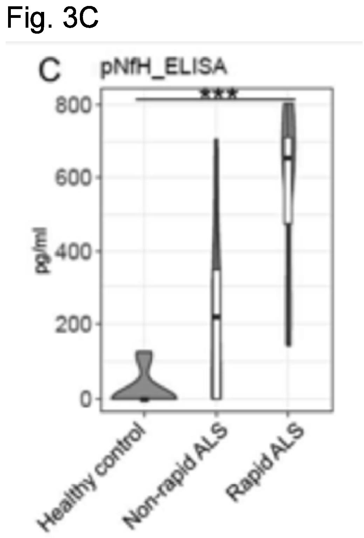

[Figure 3] Serum immunoproteomics. A) Volcano plot showing -log10 P-value and log2 fold change by differential expression analysis between each combination. Red dots are differentially expressed proteins (DEP) and show protein names. B) Violin plots showing expression levels of six proteins isolated as DEPs in rapid-amyotrophic lateral sclerosis (ALS) compared to non-rapid-ALS. Numbers indicate the median for each group. Lines and asterisks indicate significant combinations by Tukey HSD test (*p < 0.05, **p < 0.005, and ***p < 0.0005). C) Violin plot showing expression levels of phosphorylated neurofilament H (pNf-H) measured by enzyme-linked immunosorbent assay (ELISA). IL-17A = interleukin-17A; KLD1 = killer cell lectin-like receptor D1; KRT19 = keratin 19; NCF2 = neutrophil cytosolic factor 2; NPX = normalized protein expression; TFF2 = trefoil factor 2; YTHDF3 = YTH N6-methyladenosine RNA binding protein F3.
[Figure 4] Correlation diagram between serum immune proteins and frequency/ratio of each cell type. Scatter plot of expression levels of serum proteins and frequency/ratio of each cell type in single-cell RNA sequencing analysis using Pearson's correlation coefficient. Numbers indicate correlation coefficients using 40 amyotrophic lateral sclerosis patients and healthy control samples.
[Figure 5-1] Annotation of each cluster by cell type markers.
[Figure 5-2] Annotation of each cluster by cell type markers (continued from Figure 5-1).
[Figure 6] Inter-group comparison of frequencies of each cell type in all cells.
[Figure 7] Inter-group comparison of frequencies of each cell type in each immune cell.
[Figure 8] Inter-group comparison of frequency ratios between cells.

### MODE FOR CARRYING OUT THE INVENTION

### Definitions

Unless otherwise defined, all technical and scientific terms used herein have the same meaning as commonly understood by one of ordinary skill in the art to which this invention belongs. Any methods and materials similar or equivalent to those described herein can be used in the practice or testing of the present invention. Unless otherwise indicated, the practice of the present invention may employ conventional methods of chemistry, biochemistry, molecular biology, cell biology, genetics, immunology, and pharmacology, among others, within the skill of the art.

As used herein, "subject" and "patient" include humans or any non-human animals. "Non-human animals" include, but are not limited to, non-human primates such as monkeys, vertebrates such as sheep and dogs, and rodents such as mice, rats, and guinea pigs.

As used herein, "amyotrophic lateral sclerosis (ALS)" refers to a neurodegenerative disease in which motor neurons (nerve cells) are impaired, and refers to a disease in which muscle atrophy and muscle weakness progress. As used herein, ALS includes "rapid progression rate" ALS and "non-rapid progression rate" ALS. The "progression rate" of ALS includes "rapid progression rate" of ALS and "non-rapid progression rate" of ALS.

As used herein, "rapid progression rate" ALS refers to ALS showing a decline of 1 point or more per month based on the score of the revised ALS Functional Rating Scale (ALSFRS-R) for the evaluation of ALS symptoms. As used herein, "rapidly progressive" ALS, "rapid-"ALS, and "rapid" ALS are used interchangeably.

As used herein, "non-rapid progression rate" ALS refers to ALS showing a decline of less than 1 point per month based on the score of the revised ALS Functional Rating Scale (ALSFRS-R) for the evaluation of ALS symptoms. As used herein, "non-rapidly progressive" ALS, "non-rapid-"ALS, and "non-rapid" ALS are used interchangeably.

The revised ALS Functional Rating Scale is shown in Table A.

**[Table A-1]**

| **ALSFRS-R (The revised ALS Functional Rating Scale) *Circle one number for each item*.** | | |
|---|---|---|
| **1. Speech** | 4 | Normal speech processes |
| | 3 | Detectable speech disturbance |
| | 2 | Intelligible with repeating |
| | 1 | Speech combined with nonvocal communication |
| | 0 | Loss of useful speech |
| **2. Salivation** | 4 | Normal |
| | 3 | Slight but definite excess of saliva in mouth; may have nighttime drooling |
| | 2 | Moderately excessive saliva; may have minimal drooling |
| | 1 | Marked excess of saliva with some drooling |
| | 0 | Marked drooling; requires constant tissue or handkerchief |
| **3. Swallowing** | 4 | Normal eating habits |
| | 3 | Early eating problems --- occasional choking |
| | 2 | Dietary consistency changes |
| | 1 | Needs supplemental tube feeding |
| | 0 | NPO (exclusively parenteral or enteral feeding) |
| **4. Handwriting** | 4 | Normal |
| | 3 | Slow or sloppy; all words are legible |
| | 2 | Not all words are legible |
| | 1 | Able to grip pen but unable to write |
| | 0 | Unable to grip pen |

**[Table A-2]**

| **5. Cutting food and handling utensils: Evaluate either (1) or (2) depending on presence of gastrostomy.** | | |
|---|---|---|
| **(1) (Patients without gastrostomy) Handling utensils** | | |
| | 4 | Normal |
| | 3 | Somewhat slow and clumsy, but no help needed |
| | 2 | Can use a fork and spoon but cannot use chopsticks |
| | 1 | Food must be cut by someone, but can still feed slowly with a fork or spoon |
| | 0 | Needs to be fed by somebody |

| **(2) (Patients with gastrostomy) Finger movements** | | |
|---|---|---|
| | 4 | Normal |
| | 3 | Clumsy, but able to perform all manipulation independently |
| | 2 | Some help needed with closures and fasteners |
| | 1 | Provides minimal assistance to caregiver (needs help for self-care) |
| | 0 | Unable to perform any fingertip movements |
| **6. Dressing and hygiene** | 4 | Dresses normally without difficulty |
| | 3 | Independent and complete self-care with effort or decreased efficiency |
| | 2 | Intermittent assistance or substitute methods |
| | 1 | Needs attendant for self-care |
| | 0 | Total dependence |
| **7. Turning in bed and adjusting bed clothes** | 4 | Normal function |
| | 3 | Somewhat slow and clumsy, but no help needed |
| | 2 | Can turn alone or adjust sheets, but with great difficulty |
| | 1 | Can initiate, but no turn or adjust sheets alone |
| | 0 | Helpless |

**[Table A-3]**

| | | |
|---|---|---|
| **8. Walking** | 4 | Normal |
| | 3 | Early ambulation difficulties |
| | 2 | Walks with assistance |
| | 1 | Nonambulatory functional movement |
| | 0 | Unable to move legs |
| **9. Climbing stairs** | 4 | Normal |
| | 3 | Slow |
| | 2 | Mild unsteadiness or fatigue |
| | 1 | Needs assistance |
| | 0 | Cannot do |

| **Respiratory (evaluate dyspnea, orthopnea, respiratory insufficiency)** | | |
|---|---|---|
| **10. Dyspnea** | 4 | None |
| | 3 | Occurs when walking |
| | 2 | Occurs with one or more of the following: eating, bathing, or dressing |
| | 1 | Occurs at rest, difficulty breathing when either sitting or lying |
| | 0 | Significant difficulty, considering using mechanical respiratory support |
| **11. Orthopnea** | 4 | None |
| | 3 | Some difficulty sleeping at night due to shortness of breath |
| | 2 | Needs extra pillows in order to sleep |
| | 1 | Can only sleep when sitting up |
| | 0 | Unable to sleep |
| **12. Respiratory insufficiency** | 4 | None |
| | 3 | Intermittent use of breathing support device (such as BiPAP) |
| | 2 | Continuous use of breathing support device (such as BiPAP) during the night |
| | 1 | Continuous use of breathing support device (such as BiPAP) all day (during the night and day) |
| | 0 | Invasive mechanical ventilation by intubation or tracheostomy |
| **Total Score** | | **(Maximum 48 points)** |

As used herein, "likelihood of disease" refers to the possibility of having ALS or the possibility of having developed ALS. "Likelihood of disease" includes high "likelihood of disease" and low "likelihood of disease". High "likelihood of disease" refers to a high possibility of having ALS or a high possibility of having developed ALS compared to a subject without ALS, a subject who will not develop ALS, or a healthy control. Low "likelihood of disease" refers to a low possibility of having ALS or a low possibility of having developed ALS, or a similar level thereof, compared to a subject without ALS, a subject who will not develop ALS, or a healthy control.

As used herein, "therapeutic agent" includes any drug used to treat, prevent, or diagnose ALS. The therapeutic agent may be a known drug for treating, preventing, or diagnosing ALS, or an unknown drug for treating, preventing, or diagnosing ALS.

As used herein, "biomarker" refers to an indicator associated with ALS, for example, an indicator for use in determining or diagnosing the progression rate or likelihood of ALS, and/or in selecting or predicting a therapeutic agent. As used herein, a biomarker includes any substance present in a sample that can be measured, for example cells (e.g., immune cells, e.g., T cells, B cells), polypeptides (e.g., immunoglobulins, peptides), polynucleotides (e.g., mRNA, DNA), and/or metabolites thereof.

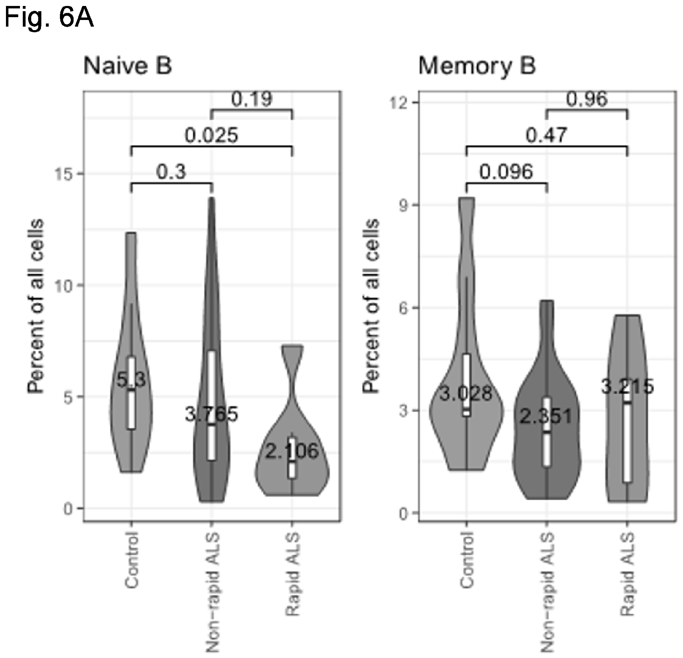

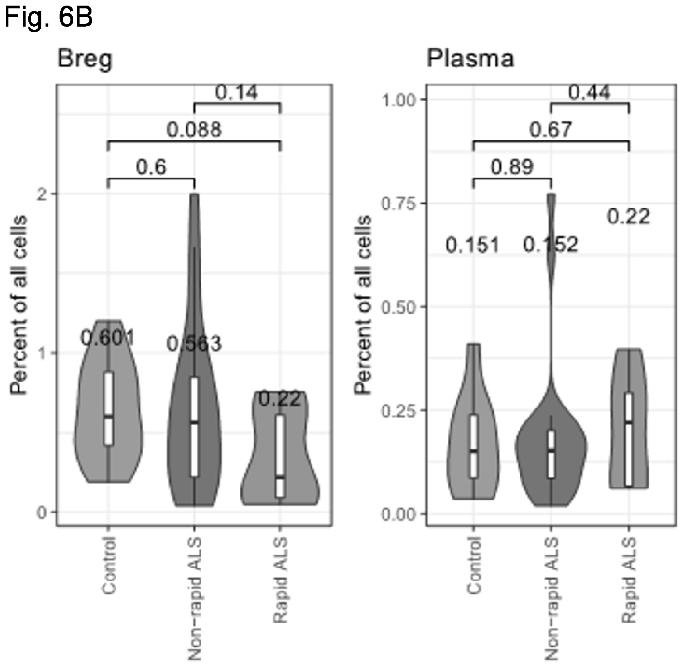

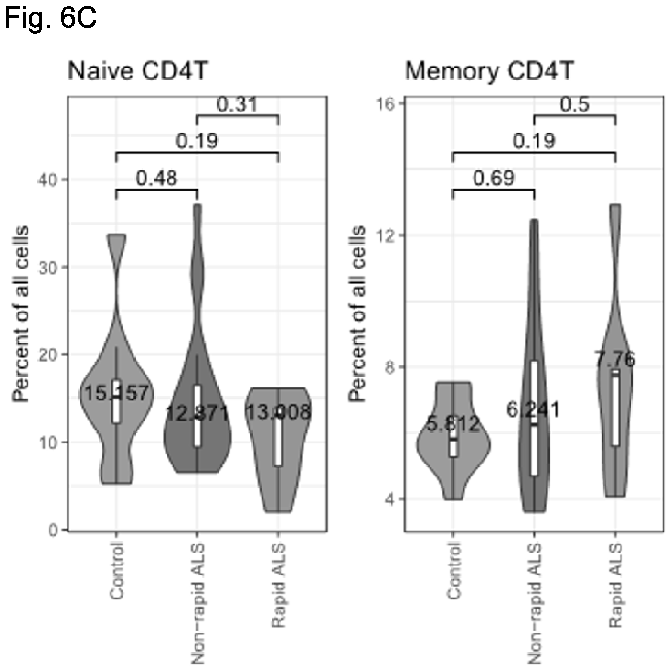

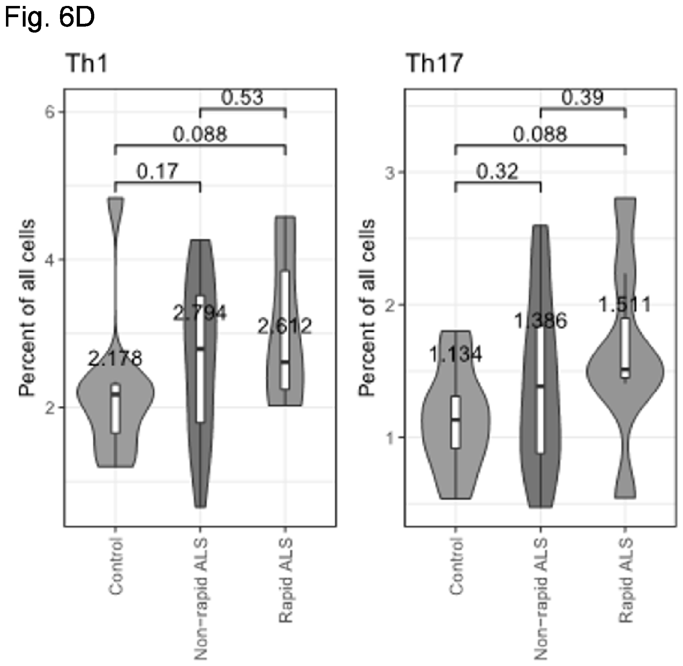

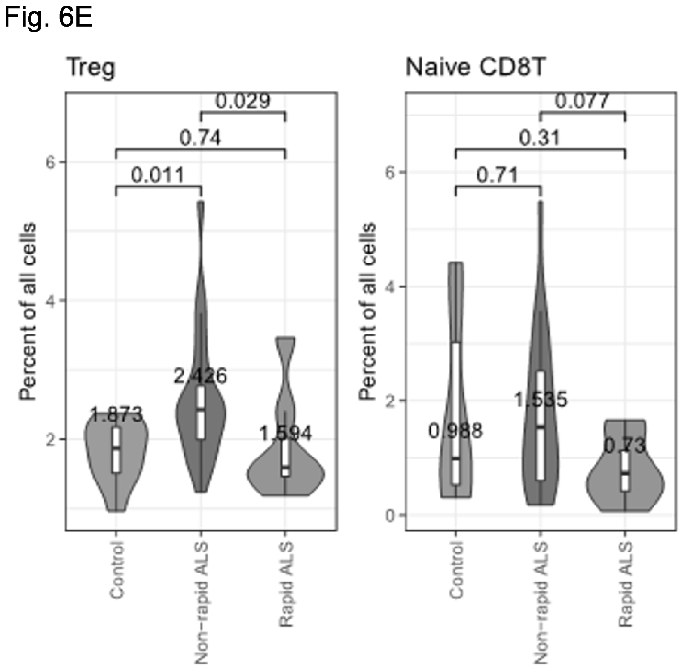

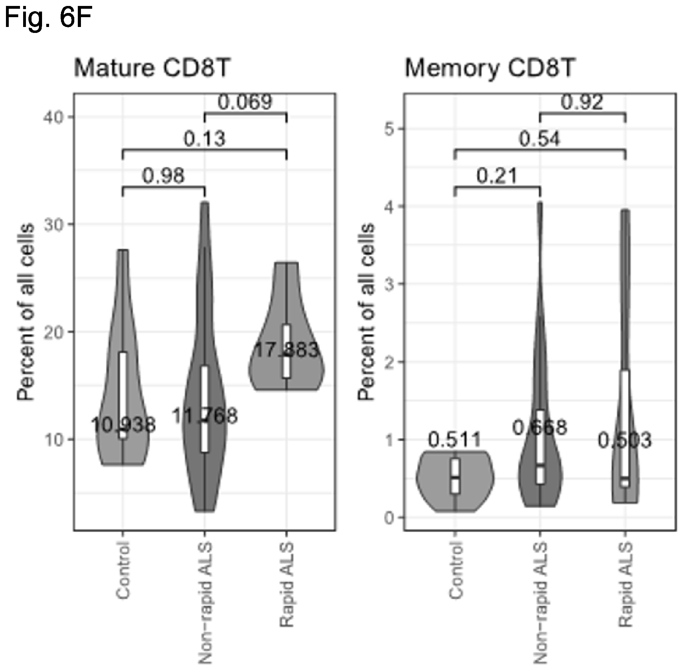

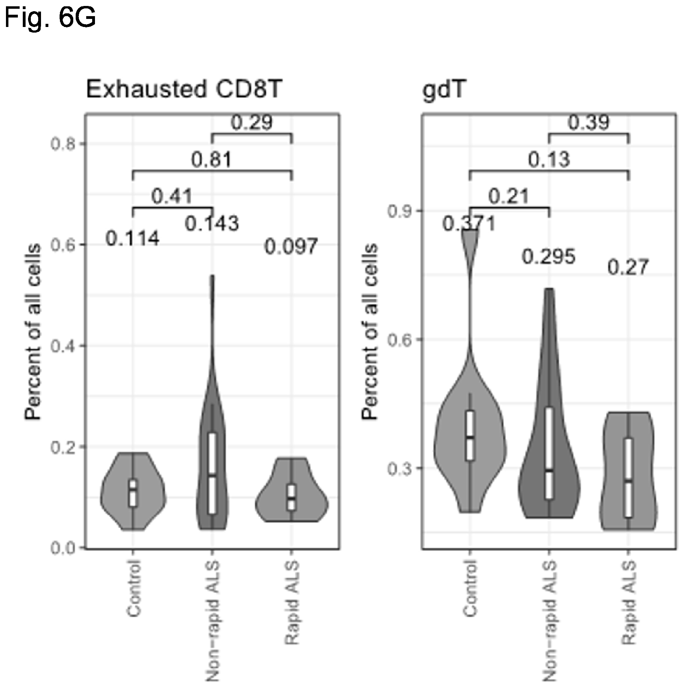

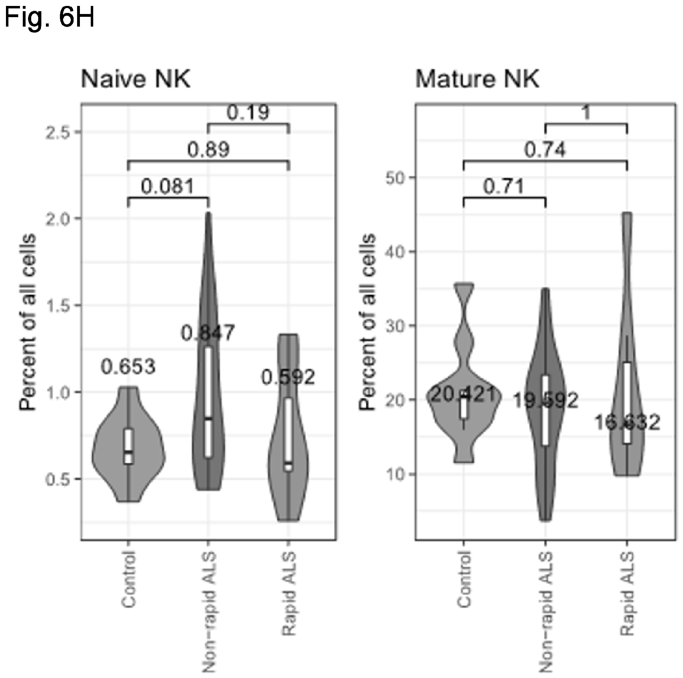

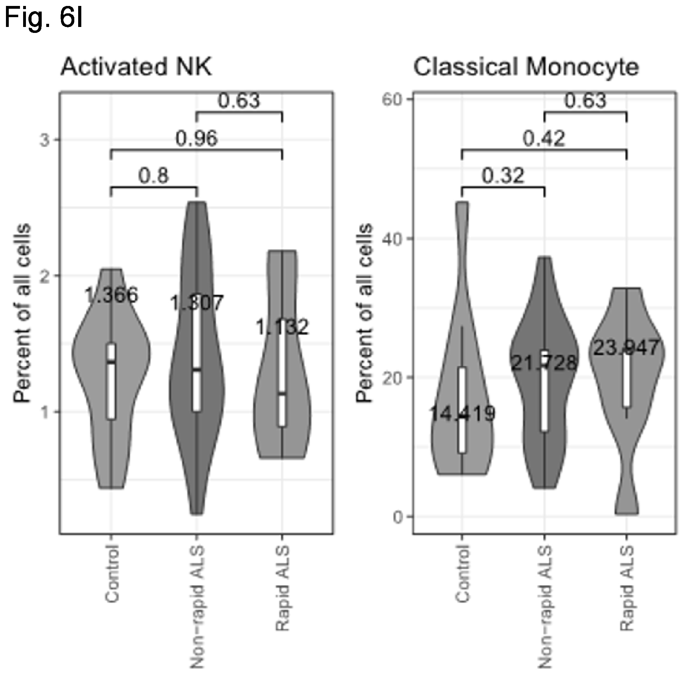

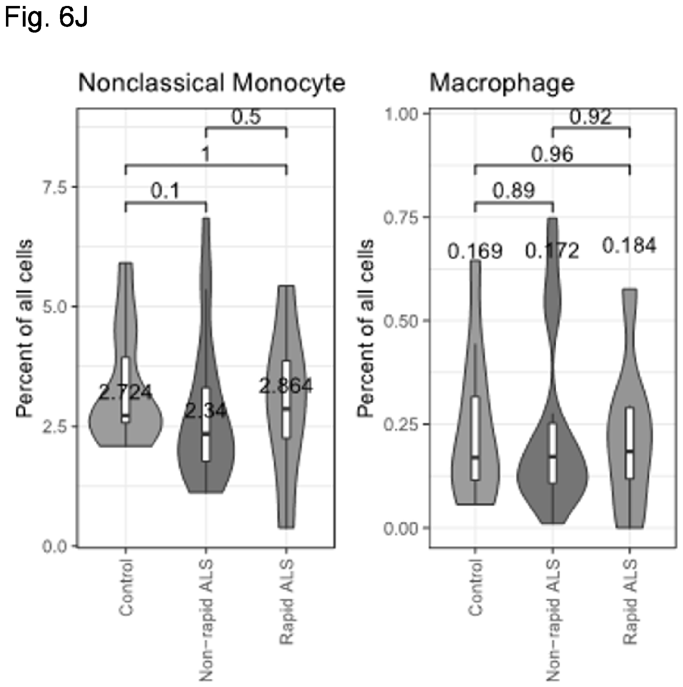

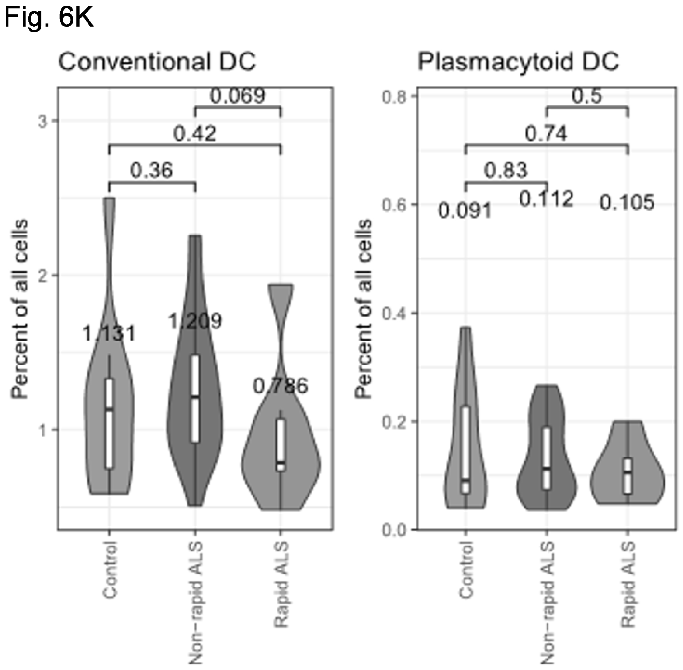

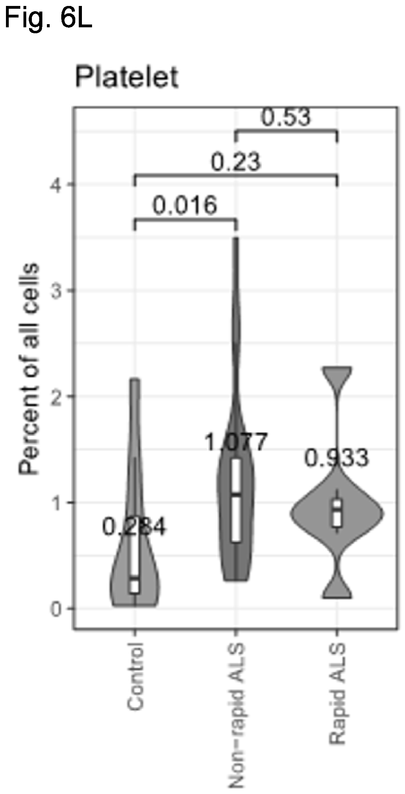

Biomarkers are described, for example, in Table 2, Table 4, Table 5, Table 6, Table 7, Table 8 (Tables 8-1, 8-2), Table 9 (Tables 9-1, 9-2, 9-3, 9-4, 9-5), Figure 2, Figure 3, Figure 4, Figure 5 (Figures 5-1, 5-2), Figure 6, Figure 7, and Figure 8. Biomarkers include biomarkers associated with ALS, biomarkers for ALS, for example, biomarkers for rapidly progressive ALS and/or biomarkers for non-rapidly progressive ALS.

A biomarker may be a single molecule or multiple molecules. When a biomarker is multiple molecules, the biomarker may be an indication of the relationship (e.g., ratio) between molecules. As used herein, the notation "single molecule A/single molecule B" of multiple molecules indicates the ratio of single molecule A to single molecule B. For example, Th17/Treg represents the ratio of the levels of Th17 and Treg by dividing the level of Th17 by the level of Treg.

A biomarker may be used as a single biomarker or in combination with multiple (e.g., two, three, four, five, six, seven, eight, or nine) biomarkers. For example, the use of multiple biomarkers can provide a more accurate or higher precision indicator associated with ALS than the use of a single biomarker.

As used herein, the "level" of a biomarker refers to the concentration, expression level, and/or activity level of the biomarker. Those skilled in the art can appropriately determine the level of a biomarker.

As used herein, "IL-17A" refers to Interleukin-17A.

As used herein, "KLRD1" refers to killer cell lectin-like receptor D1.

As used herein, "KRT19" refers to keratin 19.

As used herein, "NCF2" refers to neutrophil cytosolic factor 2.

As used herein, "TFF2" refers to trefoil factor 2.

As used herein, "YTHDF3" refers to YTH N6-methyladenosine RNA binding protein F3.

As used herein, "Th17" refers to helper T17 cells.

As used herein, "Treg" refers to regulatory T cells.

As used herein, "mature CD8 T" refers to mature CD8-positive T cells.

As used herein, "naive CD8 T" refers to naive CD8-positive T cells.

As used herein, "exhausted CD8 T" refers to exhausted CD8-positive T cells.

As used herein, "classical monocyte" refers to classical monocytes.

As used herein, "memory CD4 T" refers to memory CD4-positive T cells.

As used herein, a "sample" can be obtained from a subject or patient. A sample can be obtained from, but is not limited to, any source known in the art, such as blood, whole blood, serum, plasma, urine, interstitial fluid, tears, saliva, or skin.

As used herein, a "reference level" of a biomarker refers to the level of the biomarker in a sample derived from a subject without ALS, a sample derived from a subject with ALS, a sample derived from a subject with non-rapidly progressive ALS, or a sample derived from a subject with rapidly progressive ALS. Those skilled in the art can appropriately select and determine the reference level of a biomarker.

As used herein, the subject or patient from whom the sample is obtained may be the same as or different from the subject from whom the sample providing the reference level is obtained.

In one aspect, the biomarker of the present invention can be used to determine or diagnose the progression rate or likelihood of ALS.

In one aspect, the present invention is based on the finding that (i) the level of a biomarker in a sample obtained from a subject with rapidly progressive ALS, (ii) the level of the same biomarker in a sample obtained from a subject with non-rapidly progressive ALS, and (iii) the level of the same biomarker in a sample obtained from a subject without ALS are different.

In one aspect, the present invention is based on the finding that (i) the level of a biomarker in a sample obtained from a subject with rapidly progressive ALS, and (ii) the level of the same biomarker in a sample obtained from a subject with non-rapidly progressive ALS and/or (iii) the level of the same biomarker in a sample obtained from a subject without ALS are different.

In one aspect, the present invention is based on the finding that (ii) the level of a biomarker in a sample obtained from a subject with non-rapidly progressive ALS, and (i) the level of the same biomarker in a sample obtained from a subject with rapidly progressive ALS and/or (iii) the level of the same biomarker in a sample obtained from a subject without ALS are different.

In one aspect, the present invention is based on the finding that (i) the level of a biomarker in a sample obtained from a subject with rapidly progressive ALS and/or (ii) the level of the same biomarker in a sample obtained from a subject with non-rapidly progressive ALS, and (iii) the level of the same biomarker in a sample obtained from a subject without ALS are different.

In one aspect, the present invention provides a biomarker wherein (i) the level of the biomarker in a sample obtained from a subject with rapidly progressive ALS is higher or lower than (ii) the level of the same biomarker in a sample obtained from a subject with non-rapidly progressive ALS and/or (iii) the level of the same biomarker in a sample obtained from a subject without ALS.

In one aspect, the present invention provides a biomarker wherein (ii) the level of the biomarker in a sample obtained from a subject with non-rapidly progressive ALS is higher or lower than (i) the level of the same biomarker in a sample obtained from a subject with rapidly progressive ALS and/or (iii) the level of the same biomarker in a sample obtained from a subject without ALS.

In one aspect, the present invention provides a biomarker wherein (i) the level of the biomarker in a sample obtained from a subject with rapidly progressive ALS and/or (ii) the level of the same biomarker in a sample obtained from a subject with non-rapidly progressive ALS is higher or lower than (iii) the level of the same biomarker in a sample obtained from a subject without ALS.

In one aspect, the biomarker of the present invention can be used to select or predict a therapeutic agent for ALS.

In one aspect, a therapeutic agent that reduces or increases, respectively, (i) the level of the biomarker in a sample obtained from a subject with rapidly progressive ALS that is higher or lower than (ii) the level of the same biomarker in a sample obtained from a subject with non-rapidly progressive ALS and/or (iii) the level of the same biomarker in a sample obtained from a subject without ALS, can be selected or predicted as a possible or effective therapeutic agent for rapidly progressive ALS.

In one aspect, a therapeutic agent that reduces or increases, respectively, (ii) the level of the biomarker in a sample obtained from a subject with non-rapidly progressive ALS that is higher or lower than (i) the level of the same biomarker in a sample obtained from a subject with rapidly progressive ALS and/or (iii) the level of the same biomarker in a sample obtained from a subject without ALS, can be selected or predicted as a possible or effective therapeutic agent for non-rapidly progressive ALS.

In one aspect, a therapeutic agent that reduces or increases, respectively, (i) the level of the biomarker in a sample obtained from a subject with rapidly progressive ALS and/or (ii) the level of the same biomarker in a sample obtained from a subject with non-rapidly progressive ALS that is higher or lower than (iii) the level of the same biomarker in a sample obtained from a subject without ALS, can be selected or predicted as a possible or effective therapeutic agent for rapidly and/or non-rapidly progressive ALS.

In one aspect, when the biomarker of the present invention is used to select or predict a therapeutic agent for ALS, a candidate therapeutic agent for ALS may be mixed or contacted with the biomarker of the present invention. The candidate therapeutic agent for ALS may be a drug that reduces or increases the level of the biomarker of the present invention. The candidate therapeutic agent for ALS may be tested or confirmed to reduce or increase the level of the biomarker of the present invention.

In one aspect, the biomarker of the present invention can be used to determine or predict the efficacy or performance of a therapeutic agent for ALS.

In one aspect, the biomarker of the present invention is described, for example, in Table 2, Table 4, Table 5, Table 6, Table 7, Table 8 (Tables 8-1, 8-2), Table 9 (Tables 9-1, 9-2, 9-3, 9-4, 9-5), Figure 2, Figure 3, Figure 4, Figure 5 (Figures 5-1, 5-2), Figure 6, Figure 7, and Figure 8. In one aspect, the biomarker of the present invention is selected from the group consisting of IL-17A, KLRD1, KRT19, NCF2, TFF2, YTHDF3, Th17, regulatory T cells (Treg), mature CD8 T, naive CD8 T, exhausted CD8 T, classical monocyte, memory CD4 T, Th17/Treg, mature CD8 T/naive CD8 T, and mature CD8 T/exhausted CD8 T. In one aspect, the biomarker of the present invention is selected from the group consisting of NCF2-classical monocyte, TFF2-mature CD8 T cells, KLRD1-mature CD8 T cells, IL-17A-memory CD4 T cells, and IL-17A-Th17. In one aspect, the biomarker of the present invention is selected from the group consisting of TFF2-mature CD8 T cells, KLRD1-mature CD8 T cells, and IL-17A-Th17. In one aspect, the biomarker of the present invention is selected based on partial correlation coefficient and/or P-value, for example, as described in the Examples. In one aspect, the biomarker of the present invention may be a combination of immune cells and proteins. In one aspect, the biomarker of the present invention can be used to determine or diagnose the progression rate (rapid or non-rapid) or likelihood (high likelihood or low likelihood) of ALS. In one aspect, the biomarker of the present invention can be used to determine or diagnose the progression rate (rapid or non-rapid) or likelihood (high likelihood or low likelihood) of ALS based on an increase or decrease from a reference level.

In one aspect, the biomarker of the present invention may be used in combination with known biomarkers (e.g., neurofilament light chain (NfL)).

In one aspect, the biomarker of the present invention enables early determination or diagnosis of the progression rate or likelihood of ALS. ALS patients are generally treated after a 12-week observation period. However, the biomarker of the present invention enables early (before the 12-week observation period) determination or diagnosis of the progression rate or likelihood of ALS. Early determination or diagnosis of the progression rate or likelihood of ALS enables early treatment of ALS and may help delay ALS progression.

In one aspect, the use or method of using the biomarker of the present invention comprises a method for determining or diagnosing the progression rate or likelihood of amyotrophic lateral sclerosis (ALS) in a subject, comprising determining the level of the biomarker of the present invention in a sample derived from said subject.

In one aspect, the use or method of using the biomarker of the present invention comprises determining or diagnosing the progression rate or likelihood of ALS based on or in comparison with a reference level of the biomarker.

In one aspect, the use or method of using the biomarker of the present invention comprises determining or diagnosing the progression rate (rapid or non-rapid) or likelihood (high likelihood or low likelihood) of ALS based on the level of the biomarker in the sample being increased or decreased from the reference level.

In one aspect, the present invention provides a method for determining or diagnosing the progression rate or likelihood of amyotrophic lateral sclerosis (ALS) in a subject, comprising: determining the level of a biomarker in a sample derived from said subject; comparing said level with a reference level of said biomarker; and determining or diagnosing the progression rate (rapid or non-rapid) or likelihood (high likelihood or low likelihood) of ALS based on the level of the biomarker in the sample being increased or decreased from the reference level.

In one aspect, the use or method of using the biomarker of the present invention comprises obtaining or providing a sample derived from a subject, from or to the subject.

In one aspect, the use or method of using the biomarker of the present invention comprises determining a reference level by, for example, determining the level of the biomarker in a sample derived from a subject without ALS, a sample derived from a subject with ALS, a sample derived from a subject with non-rapidly progressive ALS, or a sample derived from a subject with rapidly progressive ALS.

In one aspect, the use or method of using the biomarker of the present invention may comprise comparing the level of the biomarker in a sample derived from the subject with the level of said biomarker in a sample derived from said subject at a different time point (e.g., 1 day before, 1 week before, 1 month before, 1 year before, etc.).

In one aspect, the reference level may be predetermined (previously determined).

In one aspect, the present invention relates to a composition for use in determining or diagnosing the progression rate or likelihood of amyotrophic lateral sclerosis (ALS), and/or in selecting or predicting a therapeutic agent, comprising a biomarker of the present invention.

In one aspect, the present invention relates to a method for treating ALS, comprising administering to a patient a therapeutic agent for amyotrophic lateral sclerosis (ALS) selected in the above aspects. Therapeutic agents for ALS include, but are not limited to, riluzole and edaravone. In some embodiments, the therapeutic agent may be an antibody against a biomarker of the present invention, for example, an antibody against IL-17A or IL-17 receptor.

Unless otherwise stated, the terms used in this specification are terms commonly used in the technical field.

The present invention will be explained in more detail below with reference to examples, but the present invention is not limited to these examples.

### Examples

### Methods

### Patients and Healthy Controls

From March 29, 2021 to October 30, 2022, 36 sporadic ALS patients and 10 healthy volunteers were screened at Tokushima University Hospital. The inclusion criteria for ALS patients were those diagnosed as definite, probable, laboratory-supported probable, or possible by updated Awaji criteria, or diagnosed as ALS by Gold Coast criteria, and within 2 years from onset. The age and sex of patients and healthy subjects were matched. The exclusion criteria for ALS patients and healthy subjects are shown in Table 1. Patients found to have known causative mutations of ALS such as SOD1 were excluded from analysis in advance because their immunological profile is different from that of sporadic ALS. Rapid ALS was defined as a decrease in the revised ALS Functional Rating Scale (ALSFRS-R) of 1.0 points/month or more (ΔALSFRS-R/month ≥ 1), and non-rapid ALS was defined as a decrease in ALSFRS-R of less than 1.0 points/month (ΔALSFRS-R/month < 1). All clinical information was collected after obtaining written informed consent from patients. All study protocols were approved by the Tokushima University Hospital Life Science and Medical Research Ethics Review Committee No. 3682. The investigation was conducted according to the principles of the Declaration of Helsinki.

### Table 1. Exclusion Criteria

**[Table 1]**

| | |
|---|---|
| (1) | Patients with tracheostomy |
| (2) | Patients with chronic obstructive pulmonary disease (COPD) |
| (3) | Patients who used edaravone within 4 weeks before consent |
| (4) | Patients who used immunomodulatory drugs (steroids, immunoglobulin preparations, etc.) within 4 weeks before consent |
| (5) | Pregnant or possibly pregnant women, or lactating women |
| (6) | Patients with severe respiratory disease, cardiovascular disease, or hepatorenal disease |
| (7) | Patients with malignant tumors |
| (8) | Patients with diseases in which immune abnormalities are involved in onset (such as autoimmune diseases) |
| (9) | Patients who participated in clinical trials within 12 weeks before consent |
| (10) | Patients from whom consent was not obtained |

### Specimen Preparation

Peripheral blood samples were collected from healthy donors and ALS patients at Tokushima University Hospital. PBMC were prepared by Ficoll-Hypaque (LymphoprepTM, Serumwerk Bernburg AG, Germany) density centrifugation, washed with phosphate buffered saline (PBS), and resuspended in X-VIVOTM medium (LONZA, Basel, Switzerland) containing 5% FBS. PBMC were stored at -80°C in CELLBANKER 1 (Nippon Zenyaku Kogyo Co., Ltd., Fukushima, Japan) until library preparation. Serum samples were collected in 8.0 ml Insepack tubes (Sekisui, Tokyo, Japan), centrifuged at 3500 rpm for 10 minutes, aliquoted, and frozen at -80°C within 20 minutes after collection.

### Single-cell RNA Sequencing (scRNA-seq)

Human PBMC were thawed, washed, and resuspended in D-PBS/BSA. After filtration, cell count and viability were assessed. scRNA-seq was performed using 10x Genomics. Primary analysis used Cell Ranger, and secondary analysis used Seurat. Data normalization, variable feature selection, and integration were performed. Principal component analysis and Uniform Manifold Approximation and Projection (UMAP) were used for dimension reduction and cell clustering. Cell types were identified based on marker expression profiles. Differentially expressed genes (DEG) were identified using Wilcoxon rank-sum test. Visualization was performed using ggplot2. Detailed methods are described below.

### Immunoproteomics

400 types of human proteins related to inflammation (Table 2) were simultaneously measured using Olink Target 96 Inflammation and Olink Explore 384 Inflammation panels (Olink Proteomics, Uppsala, Sweden). Data were shown as normalized protein expression (NPX) values. Correlation tests were performed using the 'cor.test' function of the stats package in R. Detailed methods are described below.

### Table 2. List of Measured Proteins*

**[Table 2]**

| |
|---|
| 4EBP1, ACTN4, ADA, ADAM23, ADGRE2, AGER, AGRN, AGRP, ALDH3A1, AMBN, AMN, ANGPT1, ANGPTL2, ANGPTL4, ANXA11, AOC1, ARHGEF12, ARNT, ARTN, ATP5IF1, AXIN1, B4GALT1, BACH1, BANK1, BCR, BSG, BTN2A1, BTN3A2, C1QA, CASP2, CASP8, CCL11, CCL17, CCL19, CCL20, CCL21, CCL22, CCL23, CCL24, C CL25, CCL26, CCL28, CCL3, CCL4, CCL7, CCN2, CD160, CD200, CD200R1, CD22 , CD244, CD276, CD4, CD40, CD40LG, CD48, CD5, CD58, CD6, CD70, CD79B, C D83, CD84, CD8A, CDCP1, CDON, CDSN, CEACAM21, CEP164, CHRDL1, CKAP4, CKMT1A_CKMT1B, CLEC4A, CLEC4C, CLEC4D, CLEC4G, CLEC7A, CLIP2, CLSTN 2, CNTNAP2, COL9A1, COLEC12, CRELD2, CRHBP, CRIM1, CRKL, CRLF1, CSF1, CSF3, CST5, CST7, CTRC, CTSC, CTSO, CX3CL1, CXADR, CXCL1, CXCL10, CXC L11, CXCL12, CXCL14, CXCL17, CXCL3, CXCL5, CXCL6, CXCL8, CXCL9, DAG1, DAPP1, DBNL, DECR1, DFFA, DGKZ, DNER, DNPH1, DPP10, EDAR, EGF, EGLN1 , EIF4G1, EIF5A, ENAH, ENPP5, ENPP7, ENRAGE, EPCAM, EPHA1, EPO, ERBB3, ESM1, F2R, FABP1, FABP9, FASLG, FCAR, FCRL2, FCRL3, FCRL6, FGF19, FGF2, FGF21, FGF23, FGF5, FIS1, FKBP1B, Flt3L, FLT3LG, FOXO1, FST, FSTL3, FXYD 5, GAL, GALNT3, GBP2, GDNF, GLOD4, GMPR, GOPC, GZMA, GZMB, HCLS1, HE XIM1, HGF, HLADRA, HLAE, HPCAL1, HSD11B1, HSPA1A, ICA1, ICAM4, IDS, IFN G, IFNgamma, IFNGR1, IFNLR1, IKBKG, IL10, IL10RA, IL10RB, IL11, IL12B, IL12RB 1, IL13, IL15, IL15RA, IL16, IL17A, IL17C, IL17D, IL17F, IL17RB, IL18, IL18R1, IL1 A, IL1B, IL1R2, IL1RL2, IL1RN, IL2, IL20, IL20RA, IL22RA1, IL24, IL2RB, IL32, IL3 3, IL3RA, IL4, IL4R, IL5, IL5RA, IL6, IL7, IL8, IRAK1, IRAK4, ISM1, ITGA11, ITGA 6, ITGB6, ITM2A, JCHAIN, JUN, KLRB1, KLRD1, KRT19, KYNU, LAIR1, LAMA4, L AMP3, LAP.TGFbeta1, LAP3, LAT, LGALS4, LGALS9, LGMN, LHPP, LIFR, LILRB4, LRRN1, LSP1, LTA, LTBR, LTO1, LY6D, LY75, LY9, MANF, MAP2K6, MAPK9, MA TN2, MCP1, MCP2, MCP3, MCP4, MEGF10, MEPE, MERTK, METAP1D, MGMT, MI CB_MICA, MILR1, MLN, MMP1, MMP10, MPIG6B, MVK, MYO9B, MZB1, NBN, NCF2 , NCK2, NCLN, NCR1, NELL2, NFASC, NFATC1, NFATC3, NME3, NPPC, NRTN, N T3, NT5C3A, NTF3, NUB1, NUDC, OMD, OPG, OSCAR, OSM, PADI2, PAPPA, PAR P1, PCDH1, PDGFB, PDL1, PDLIM7, PGF, PIK3AP1, PKLR, PLA2G4A, PLAUR, PLX NA4, PNLIPRP2, PNPT1, PON3, PPP1R9B, PRDX3, PRDX5, PREB, PRELP, PRKAB1, PRKCQ, PROK1, PRSS8, PSIP1, PSMG3, PSPN, PTH1R, PTPN6, PTPRM, PTX3, RAB 37, RAB6A, RABGAP1L, REG4, RGS8, ROBO1, SAMD9L, SCF, SCG3, SCGB1A1, SC GB3A2, SCGN, SCRN1, SELPLG, SERPINB8, SH2D1A, SHMT1, SIGLEC1, SIGLEC10, SIRPB1, SIRT2, SIT1, SKAP2, SLAMF1, SLAMF7, SLC39A5, SMOC2, SMPDL3A, SPI NK4, SPINT2, SPON1, SPRY2, SRPK2, ST1A1, STAMBP, STX8, SULT2A1, TANK, TB C1D5, TFF2, TGFA, TGFalpha, TGFB1, TIMP3, TLR3, TNF, TNFAIP8, TNFB, TNFR SF11A, TNFRSF11B, TNFRSF13B, TNFRSF13C, TNFRSF14, TNFRSF4, TNFRSF9, TN FSF10, TNFSF11, TNFSF12, TNFSF13, TNFSF14, TPP1, TPSAB1, TPT1, TRAF2, TR AIL, TRANCE, TREM2, TRIM21, TRIM5, TWEAK, uPA, VASH1, VEGFA, VEGFD, W AS, WFIKKN2, WNT9A, YTHDF3 |

| |
|---|
| *These proteins are included in Olink Target 96 Inflammation and Olink Explore 384 Inflammation panels (https://olink.com). |

### Enzyme-Linked Immunosorbent Assay (ELISA)

Serum phosphorylated neurofilament H (pNf-H) levels were measured using a commercially available ELISA kit (EUROIMMUN, Luebeck, Germany).

### Statistical Analysis

Statistical analysis was performed using Microsoft R open source software (version 4.0.2). DEGs in scRNA-seq were identified by nonparametric Wilcoxon rank-sum test and multiple testing was corrected using Bonferroni's test. DEPs in serum proteome and differences between groups in the frequency of each cell type were identified using Tukey's range test for multiple testing. Correlation tests were performed using Pearson's correlation coefficient.

### Detailed Single-cell RNA Sequencing (scRNA-seq)

PBMC Preparation, Library Preparation, and Sequencing Cryopreserved human peripheral blood mononuclear cells (PBMC) were rapidly thawed in 37°C water and washed with 1x D-PBS (Mg/Ca-free) containing 1% BSA. Centrifugation conditions for cell recovery were 20°C, 250 xg, swing-out rotor for 10 minutes. After 3 washes, cells were resuspended in an appropriate amount of D-PBS/1% BSA and passed through a Flowmi Cell Strainer 40 µm to remove remaining large particles. Cell count and viability were examined with a hemocytometer with trypan blue staining. scRNA-seq was performed using the 10x Genomics platform. Single cell suspensions (10,000 cells) were loaded onto a GEM generation chip using a 10x Genomics Chromium controller, and DNA libraries were prepared using Chromium Next GEM Single Cell 3' Reagent Kits v3.1 (10x Genomics, Pleasanton, CA) according to the manufacturer's instructions. Quality control of the prepared libraries was performed before sequencing using 4200 TapeStation D1000 ScreenTape (Agilent, Santa Clara, CA) and Qubit dsDNA Assay (Thermo Fisher Scientific, Waltham, MA). Gene expression libraries were sequenced at a depth of 50,000 reads per cell using the DNBSEQ-G400 platform (MGI Tech, Shenzhen, China).

### Data Normalization

Primary analysis was performed using the 10x Genomics Cell Ranger version 3 pipeline. Bcl files were converted to FASTQ format using Cell Ranger 'counts' software. FASTQ data were filtered and mapped to the GRCh38 reference genome. Secondary analysis was performed using Seurat version 3 package in R version 4.0.2. Data from low-quality cells and doublet cells were excluded using Seurat and DoubletFinder packages. These counts were normalized using global scaling normalization method with Seurat function 'LogNormalize' and log-transformed with Seurat function 'NormalizeData'. Variable features were selected by direct modeling of inherent mean-variance relationships using Seurat function 'FindVariableFeatures', and these features were used to select integration features using Seurat function 'SelectIntegrationFeatures'. Integration features were applied to linear transformation and used for principal component analysis (PCA) using Seurat functions 'ScaleData' and 'RunPCA', respectively. Using reciprocal PCA, the top 50 principal components were used for anchor identification. Anchors were used to integrate datasets using Seurat function 'IntegrateData'.

### Analysis

Integrated normalized data were subjected to linear transformation and PCA using Seurat functions 'ScaleData' and 'RunPCA'. Significant principal components were determined using an elbow plot plotting the standard deviation of principal components and were adopted to visualize Uniform Manifold Approximation and Projection (UMAP) using Seurat functions 'ElbowPlot' and 'RunUMAP'. Significant principal components were also used to identify cell types. First, the similarity between each cell was calculated by a KNN graph based on Euclidean distance in PCA space and the weight of edges between any two cells based on shared overlap in local neighborhoods using Seurat function 'FindNeighbors'. Next, cells were divided into clusters by clustering the similarity between cells using Louvain algorithm and Seurat function 'FindClusters'. Finally, cell types in each cluster were identified by examining cell type-specific marker expression profiles in each cluster. Differentially expressed genes (DEG) between groups in each cell type were identified by nonparametric Wilcoxon rank-sum test using Seurat function 'FindMarkers'. Gene expression levels in each cluster were visualized in violin plots using Seurat function 'VlnPlot'. Differences in frequency of each cell type between groups were identified by Tukey's range test using 'TukeyHSD' function of stats (R default) package. Correlation tests were performed using 'cor.test' function of stats (R default) package. Visualization of scatter plots, box plots, and bar graphs was performed in R using ggplot2 package.

### Detailed Immunoproteomics

400 types of human proteins related to inflammation (Table 2) were simultaneously measured using Olink Target 96 Inflammation and Olink Explore 384 Inflammation panels (Olink Proteomics, Uppsala, Sweden). Measurements were performed based on PEA technology (https://www.olink.com). Briefly, matched pairs of antibodies linked to unique oligonucleotides (proximity probes) bind to their respective protein targets. Only when two probes are in close proximity, they hybridize to each other and double-stranded DNA is formed. Finally, complexes were detected and quantified by quantitative real-time PCR in Olink Target 96 Inflammation panel and by next-generation sequencing (NGS) in Olink Explore 384 Inflammation panel. Data were shown as normalized protein expression (NPX) values, which are arbitrary units on a log2 scale of Olink Proteomics. NPX values were calculated using CT values (quantitative real-time PCR) and matched counts (NGS). Therefore, NPX values represent relative expression, not absolute protein levels. For proteins analyzed, all items measured by NGS were selected, and for items measured only by real-time PCR, only those with 50% or more samples above the detection limit were selected. Tukey's range test was performed using 'TukeyHSD' function of stats (R default) package to identify differentially expressed proteins (DEP) between groups. Volcano plots were created in R using ggplot2 and ggrepel packages. Visualization of scatter plots and box plots was performed in R using ggplot2 package. Correlation tests were performed using 'cor.test' function of stats (R default) package.

### Results

### Clinical Characteristics

Of those screened, informed consent was obtained from 10 healthy controls and 35 ALS patients; one patient with neuropathy was not enrolled. Five ALS patients with gastric cancer (n=1), hepatocellular carcinoma (n=1), HTLV-1 (n=1), SOD1 (n=1), and lung cancer and SOD1 (n=1) were excluded. Finally, peripheral blood samples from 10 healthy controls, 23 non-rapid ALS patients (ΔALSFRS-R/month < 1), and 7 rapid ALS patients (ΔALSFRS-R/month ≥ 1) were analyzed (Table 3). ALS patients had a disease duration of 10.4 ± 6.4 months and mean ALSFRS-R of 39.9 ± 5.2 points. Clinical information was compared with scRNA-seq and proteomics data (Figure 1A).

### Table 3. Clinical Characteristics of 40 Subjects

**[Table 3]**

| | | **Healthy Controls (n=10)** | **Non-rapid ALS (n=23)** | **Rapid ALS (n=7)** |
|---|---|---|---|---|
| Age, years (mean ± SD) | | 63.6 ± 10.2 | 60.7 ± 10.8 | 69.8 ± 14.4 |
| Male, N (%) | | 7 (70.0) | 17 (73.9) | 3 (42.9) |
| ALSFRS-R at entry, mean ± SD* | | - | 41.8 ± 2.6 | 34.7 ± 7.1 |
| Duration before entry, months, mean ± SD* | | - | 12.4 ± 6.2 | 5.0 ± 2.5 |
| ΔALSFRS-R/month, mean ± SD* | | - | 0.56 ± 0.22 | 2.81 ± 1.27 |
| **Site of onset, N (%)** | | | | |
| | Bulbars | - | 8 (34.8) | 2 (28.6) |
| | Limbs | - | 14 (60.9) | 5 (71.4) |
| | Trunk | - | 1 (4.3) | 0 (0.0) |
| %FVC, mean ± SD | | - | 87.6 ± 22.7 | 61.5 ± 41.0 |
| **Cognitive status, N (%)** | | | | |
| | Normal | 10 (100.0) | 21 (91.3) | 4 (57.1) |
| | Impaired | - | 2 (8.7): FTD, 2 | 3 (42.9): FTD, 1; suspected FTD, 1; unknown etiology, 1 |

| | | | | |
|---|---|---|---|---|
| ALS = amyotrophic lateral sclerosis, ALSFRS-R = the revised ALS Functional Rating Scale, FVC = forced vital capacity, SD = standard deviation, *p < 0.05 (non-rapid ALS vs. rapid ALS, t-test). | | | | |

### scRNA-seq Analysis Revealed Changes in Immune Cells in ALS

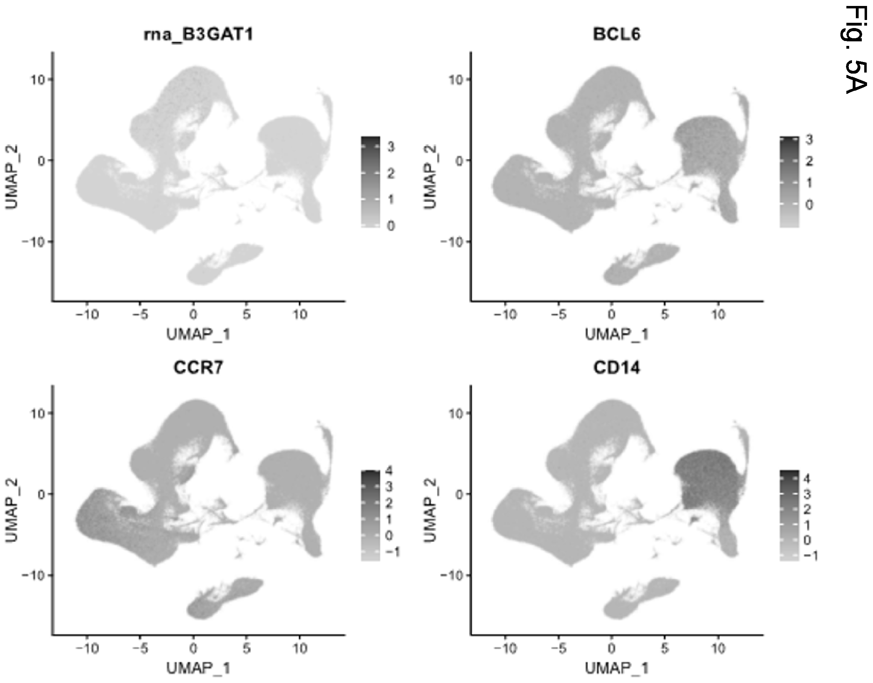

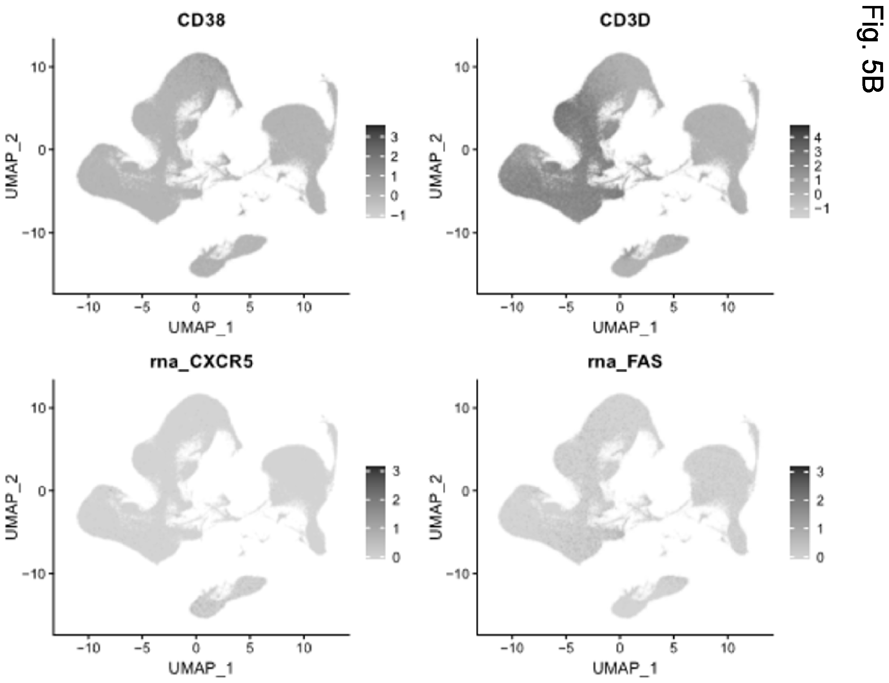

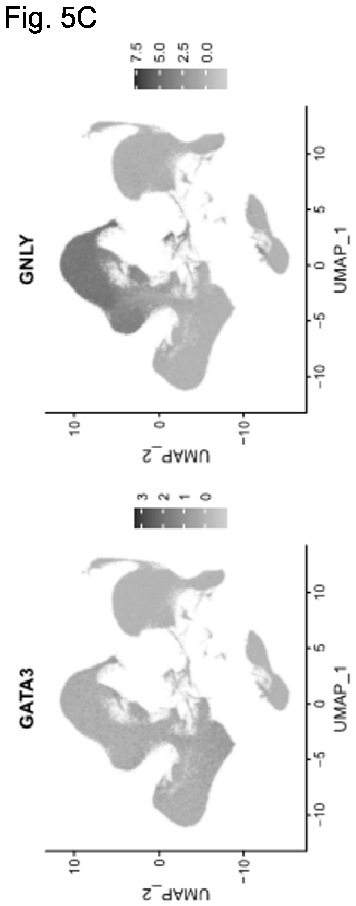

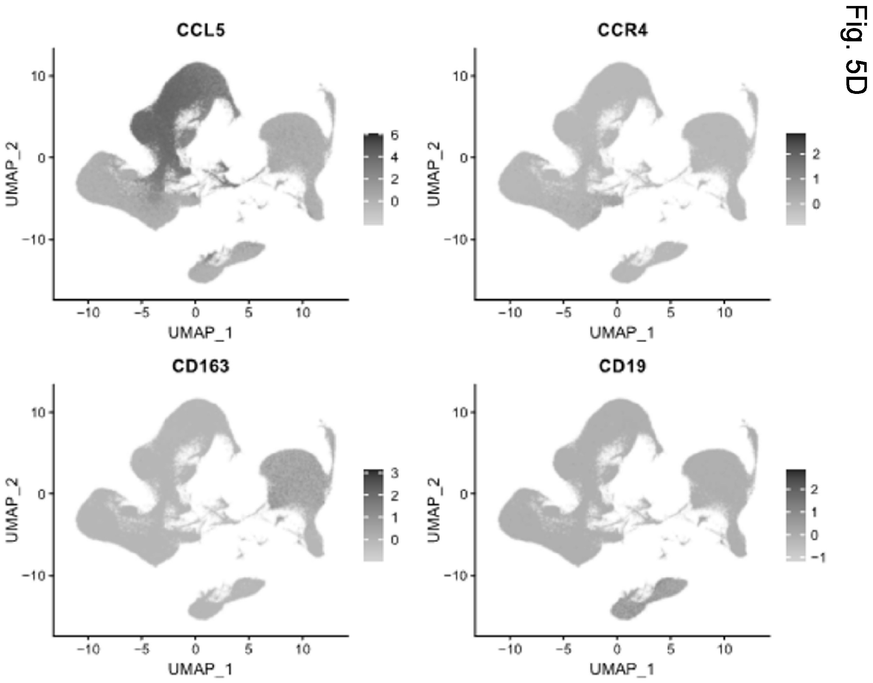

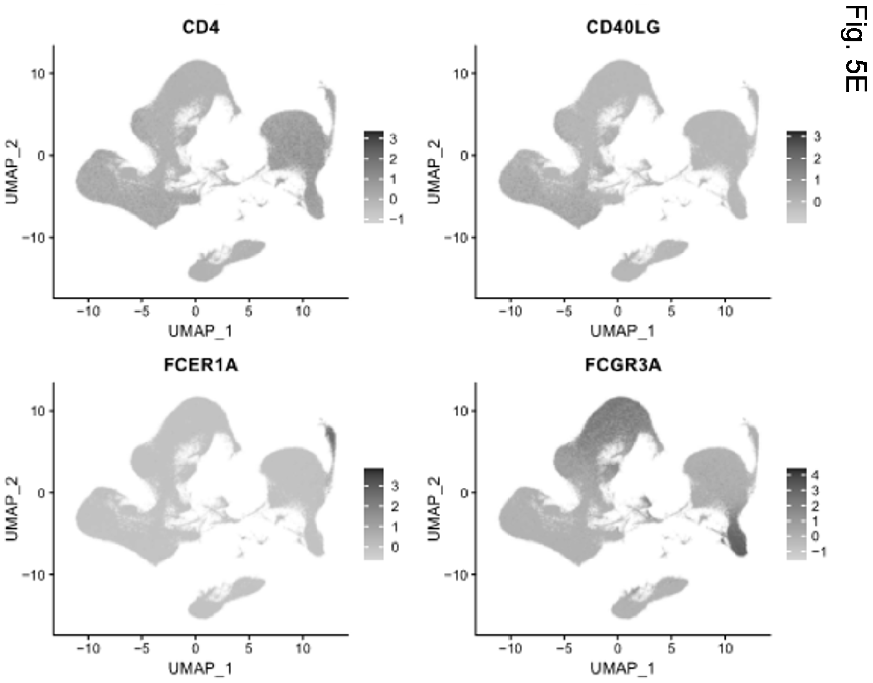

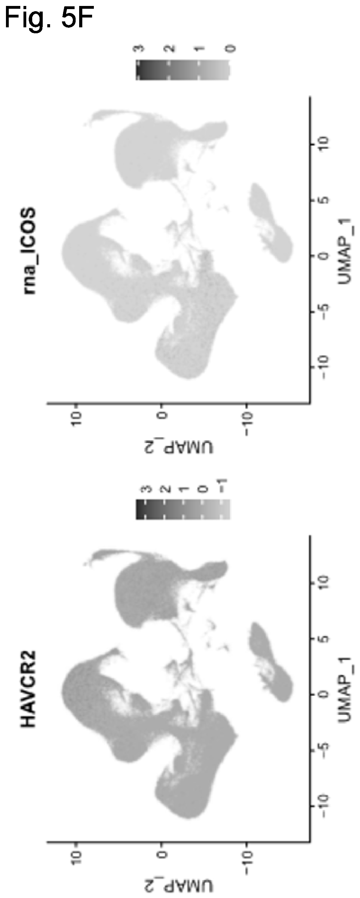

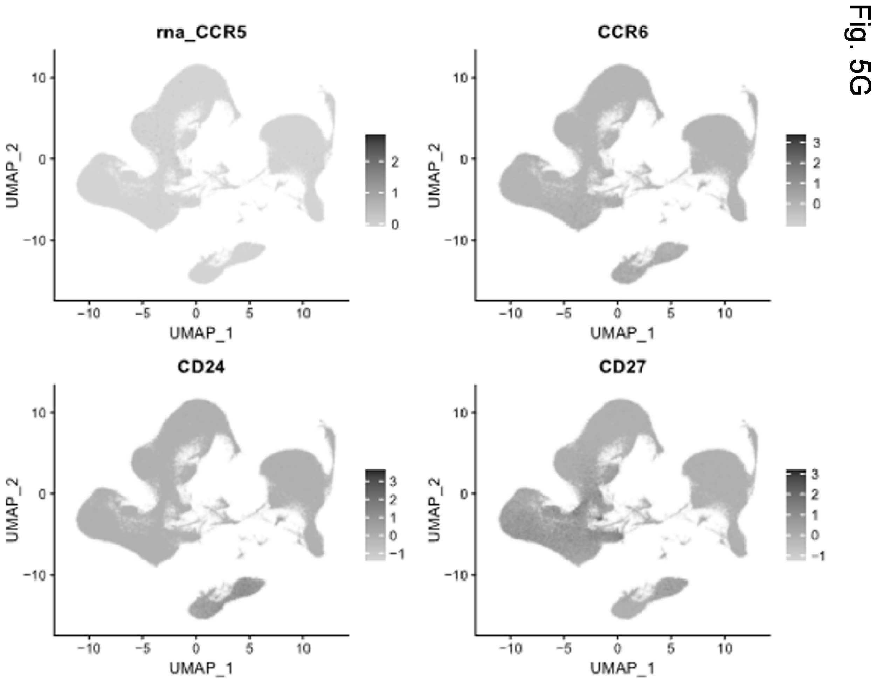

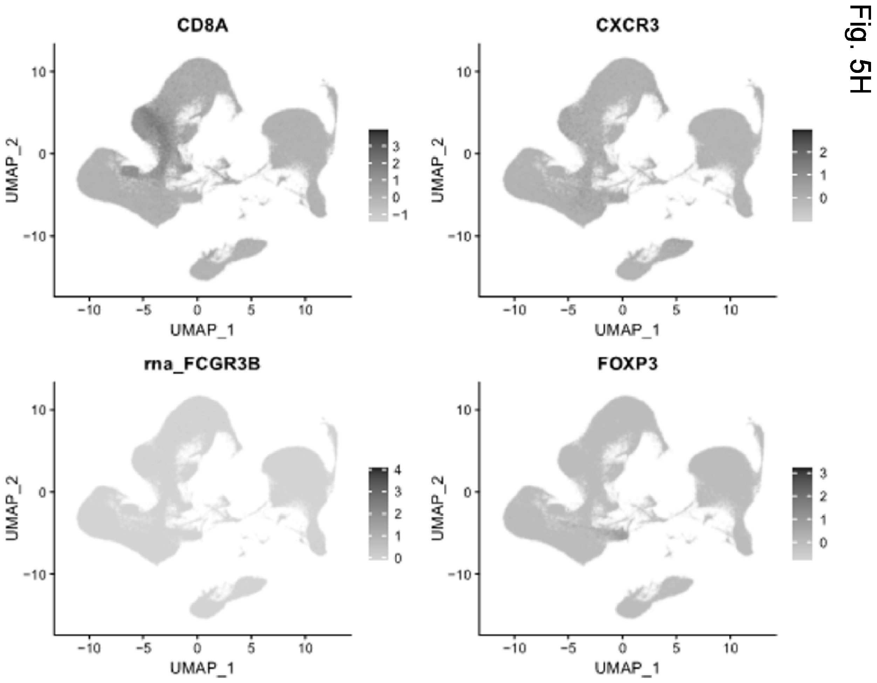

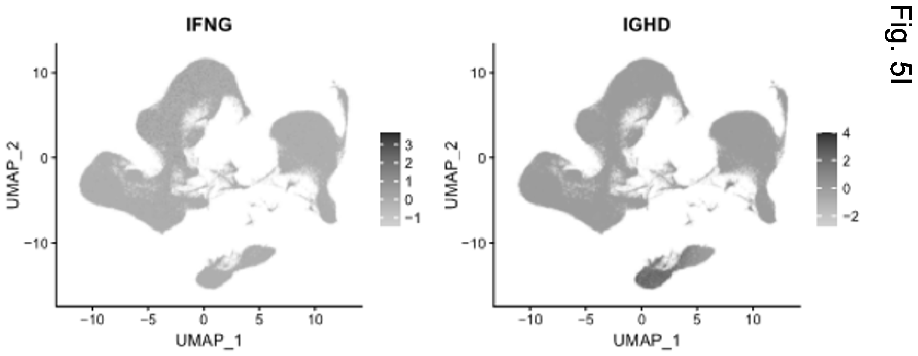

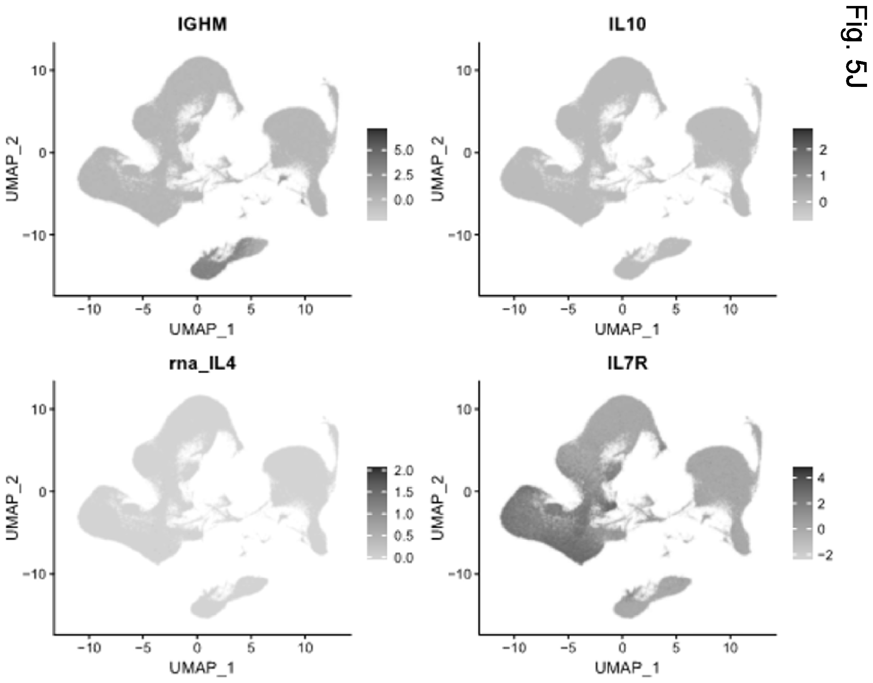

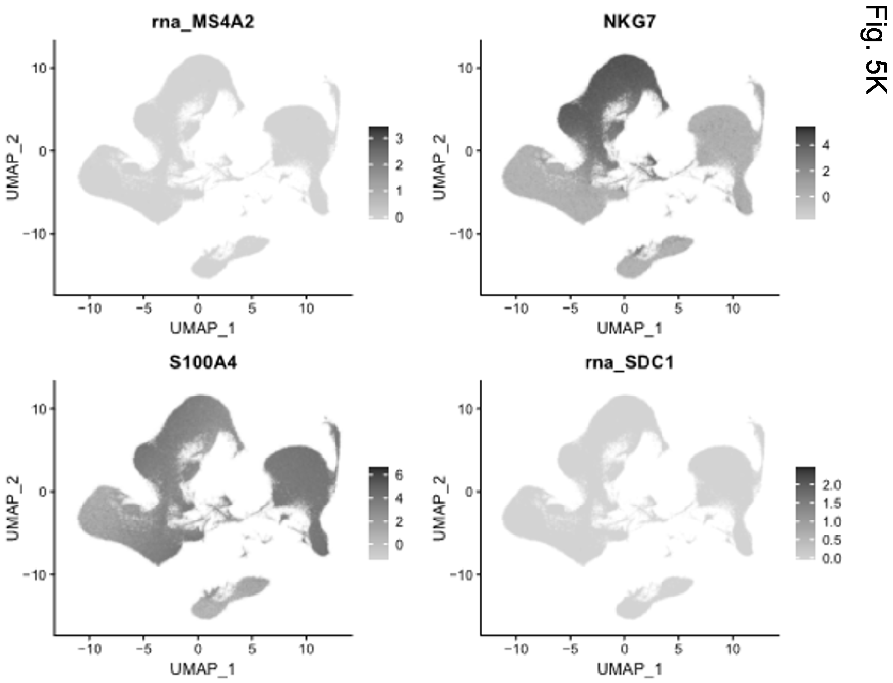

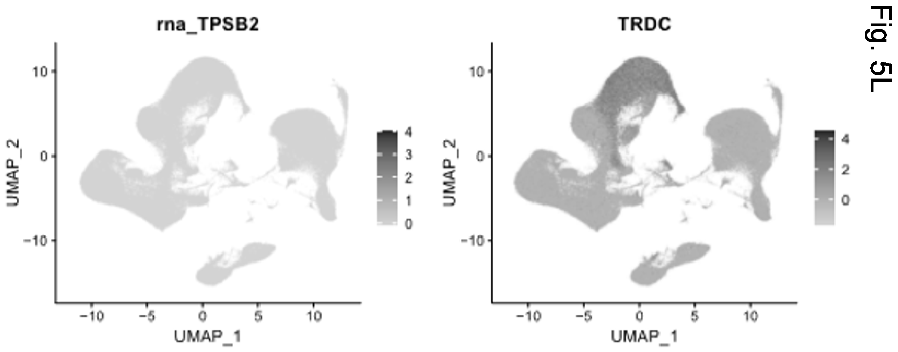

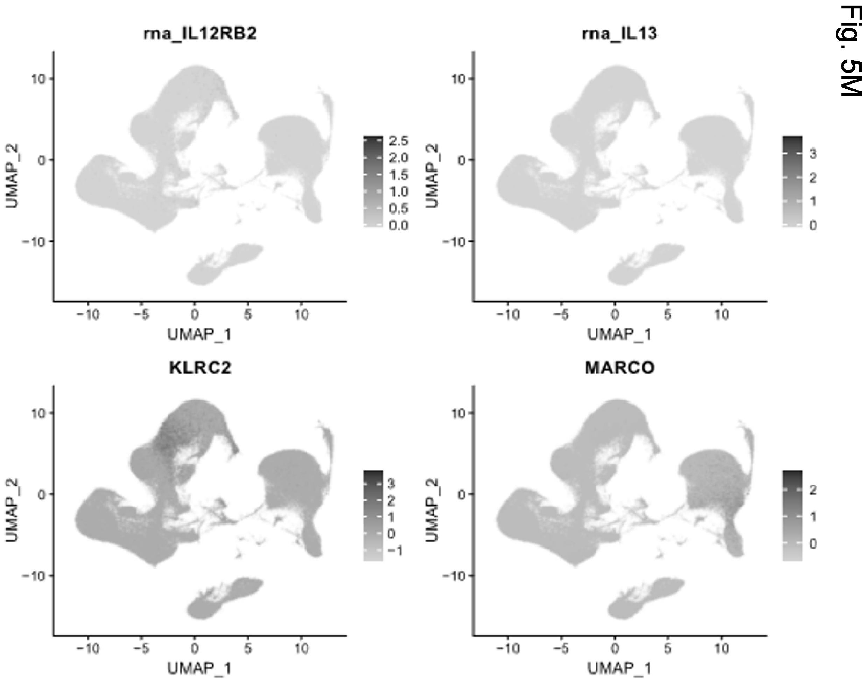

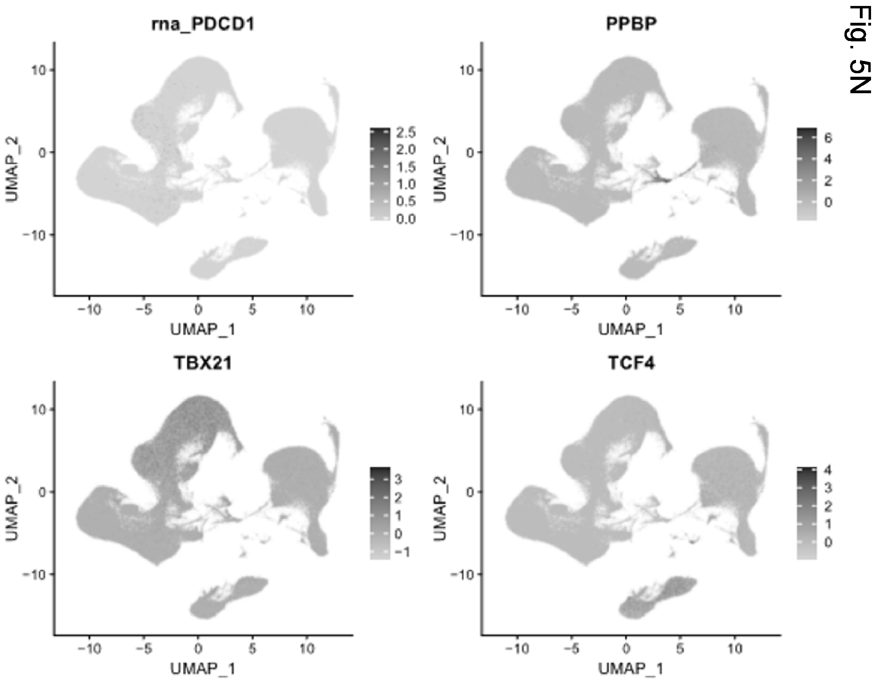

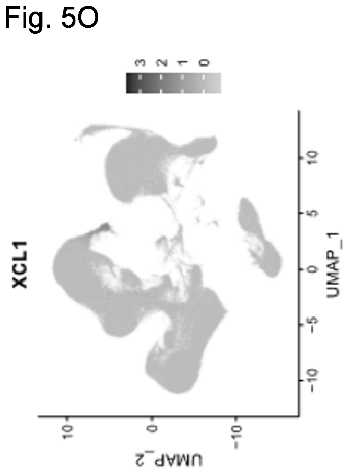

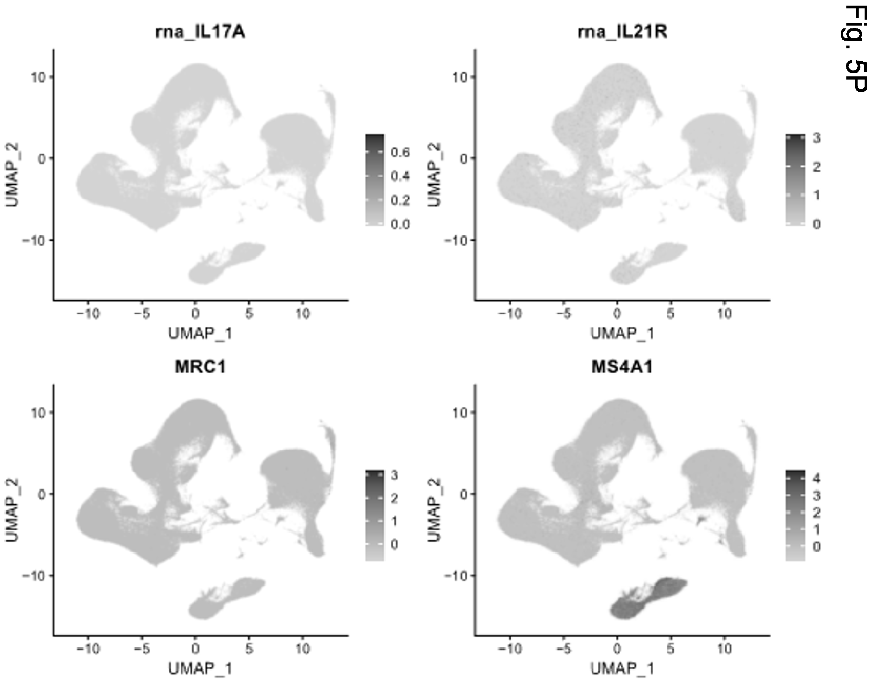

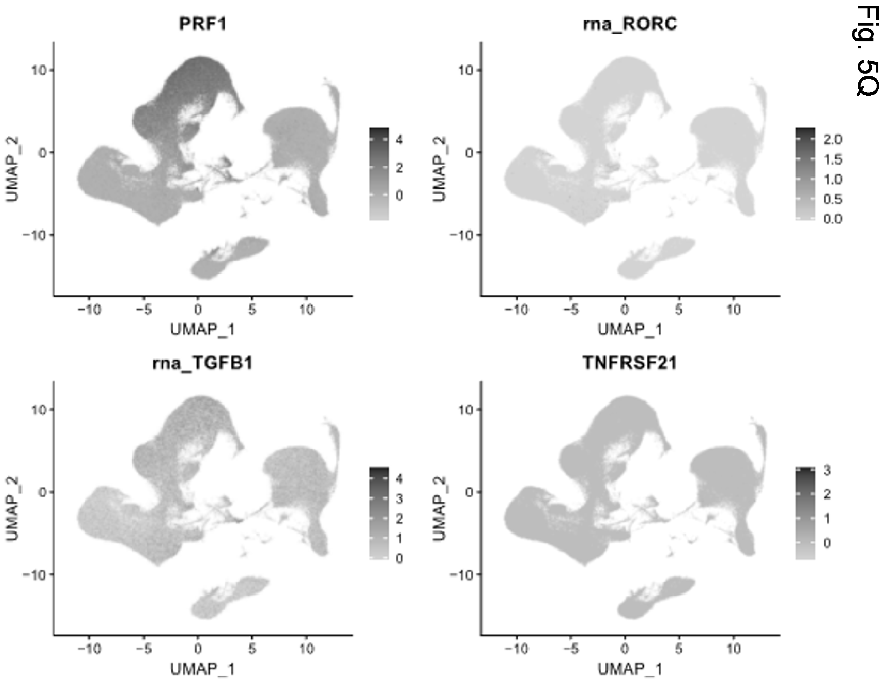

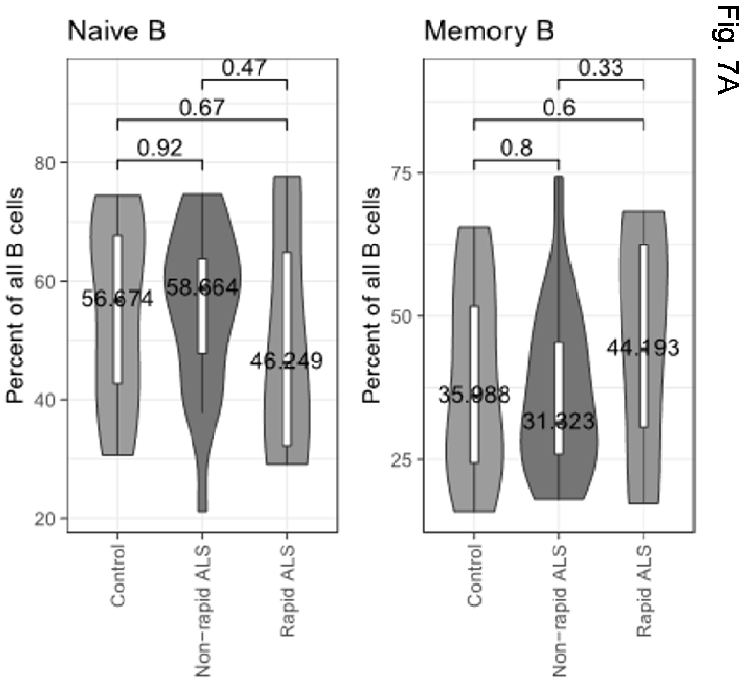

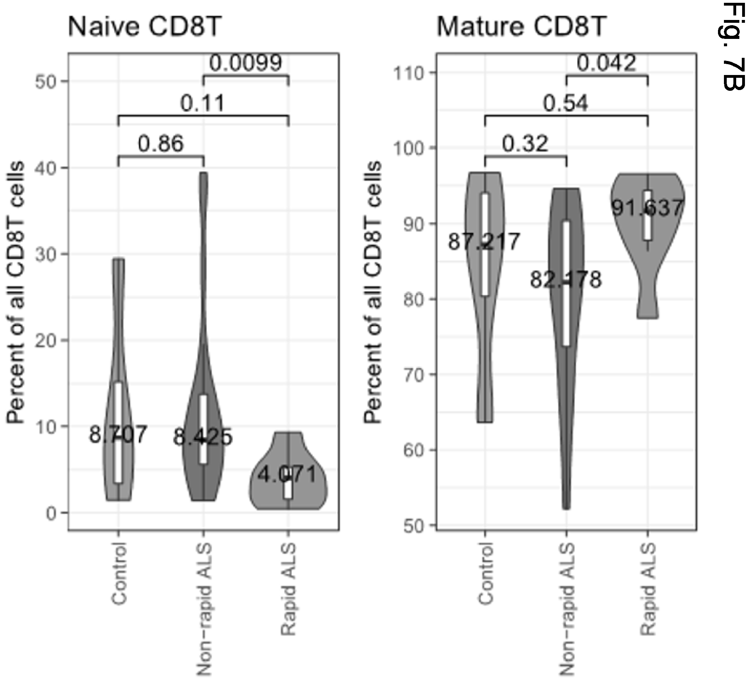

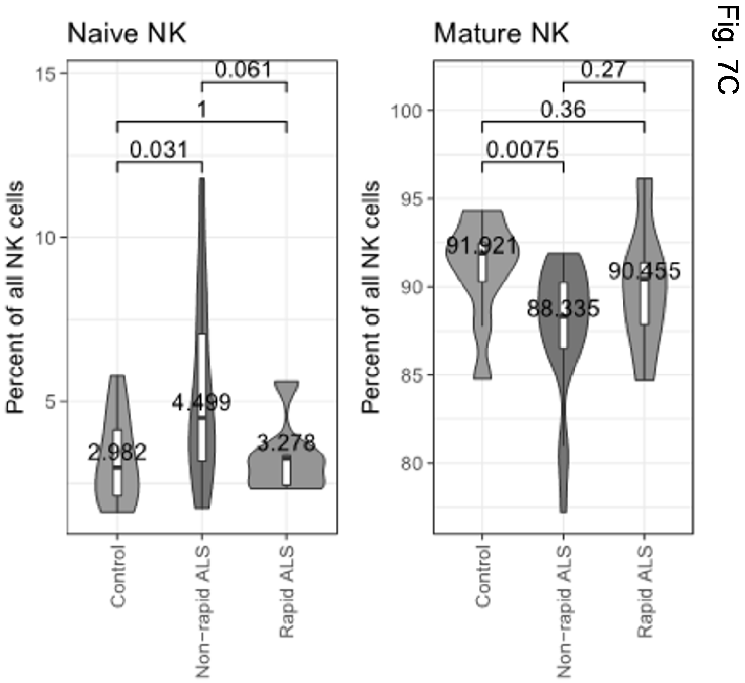

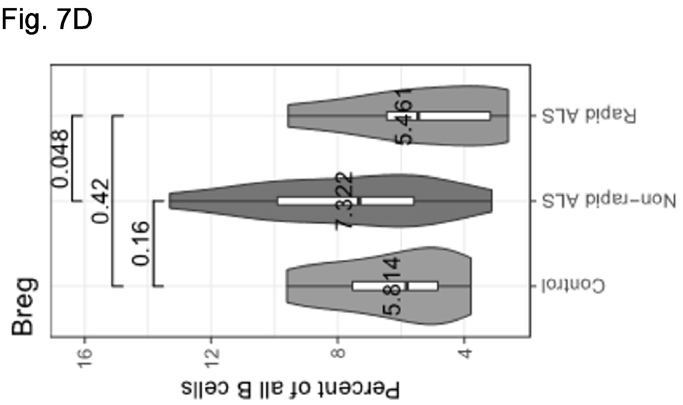

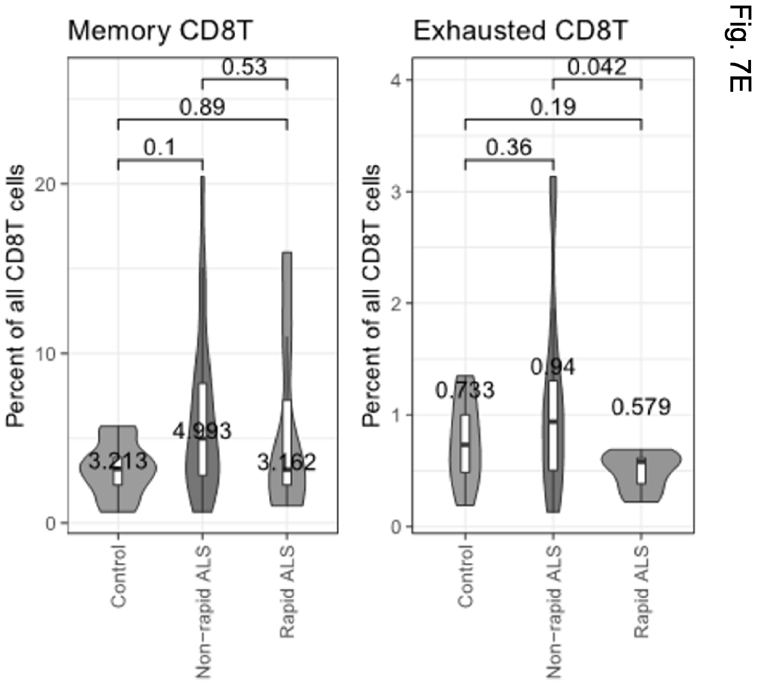

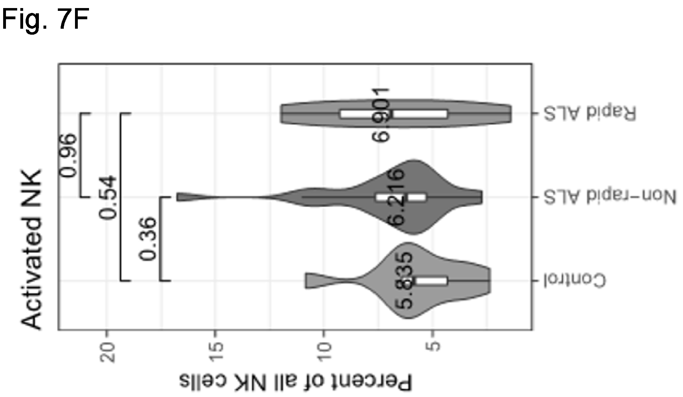

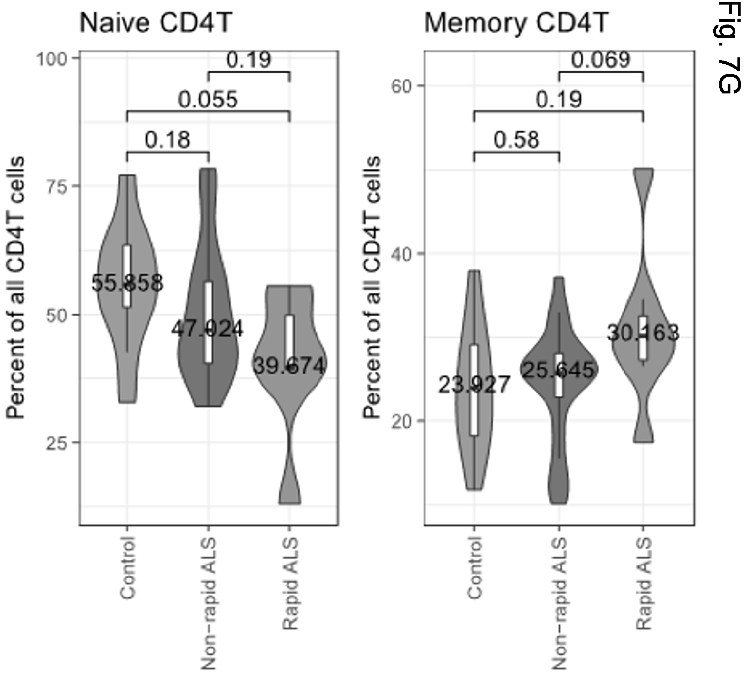

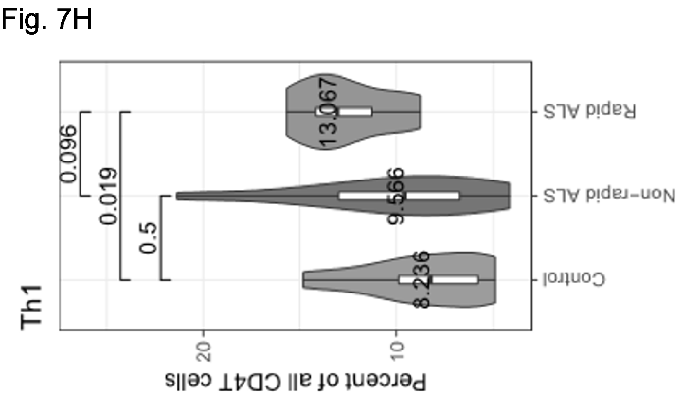

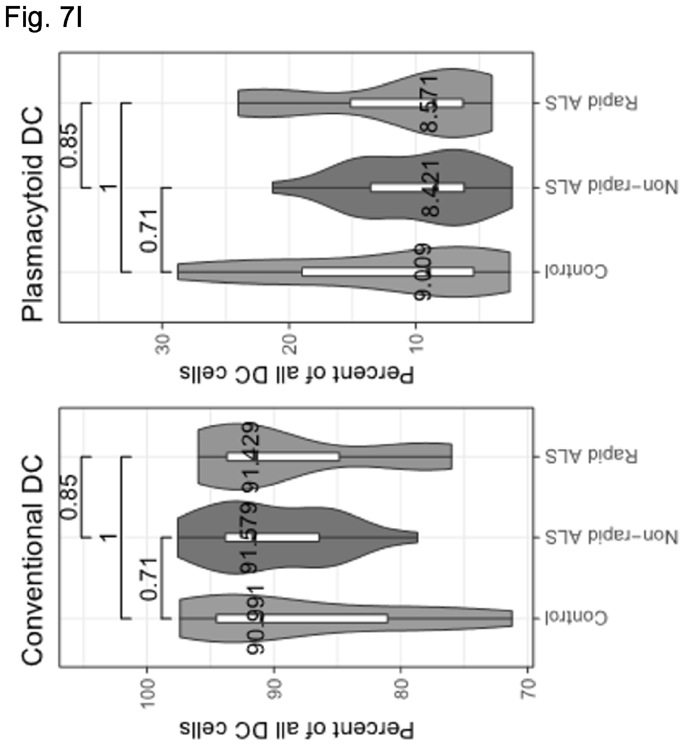

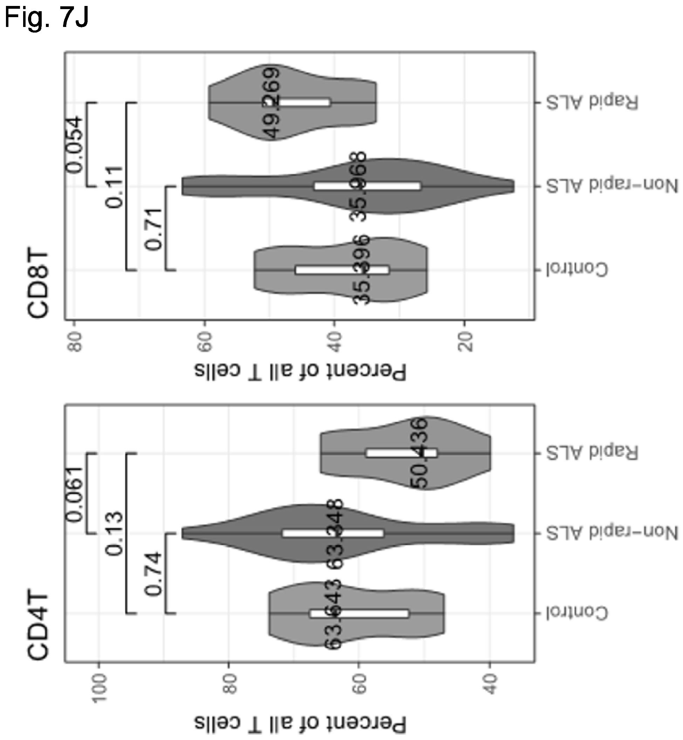

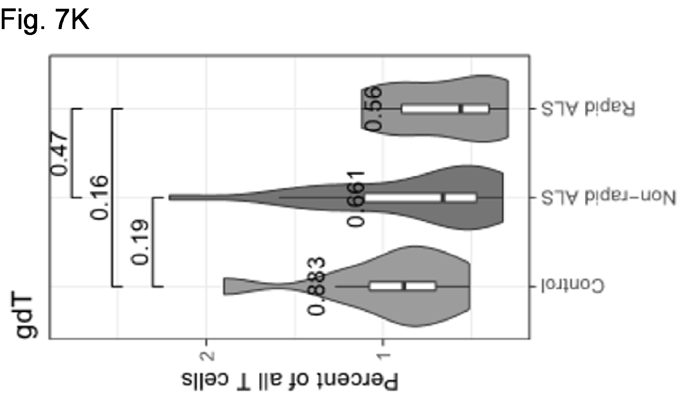

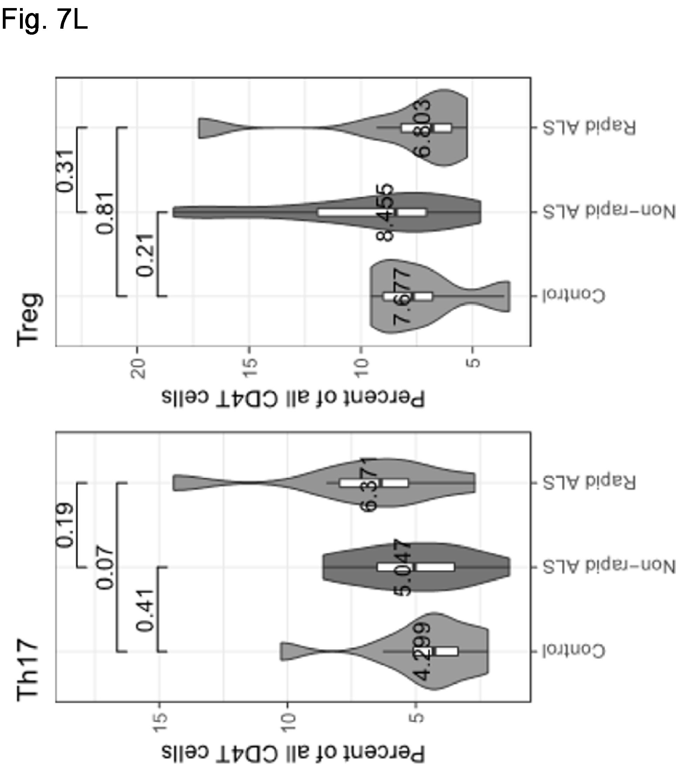

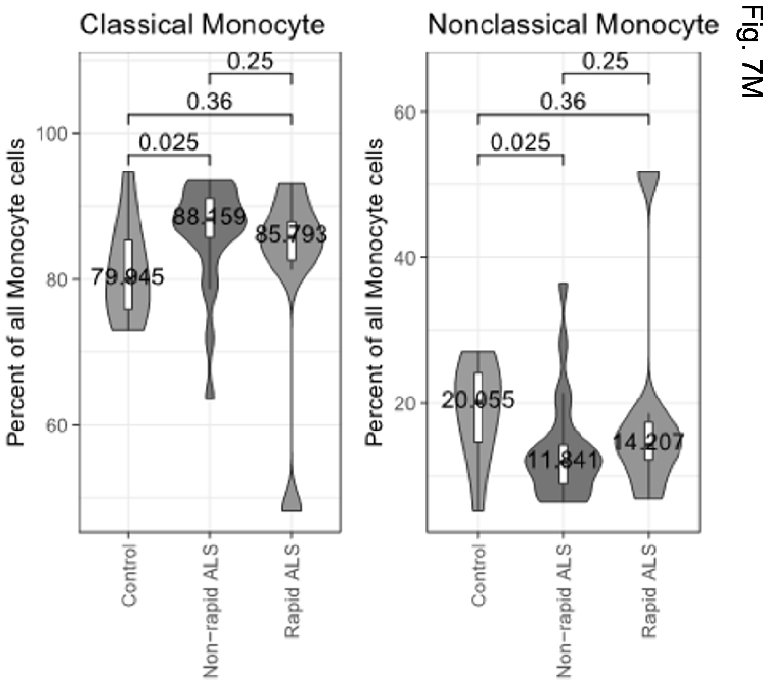

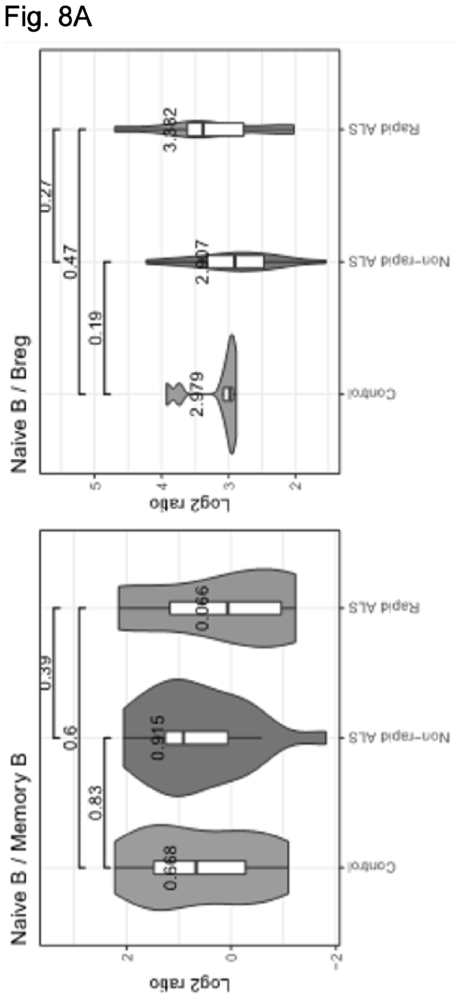

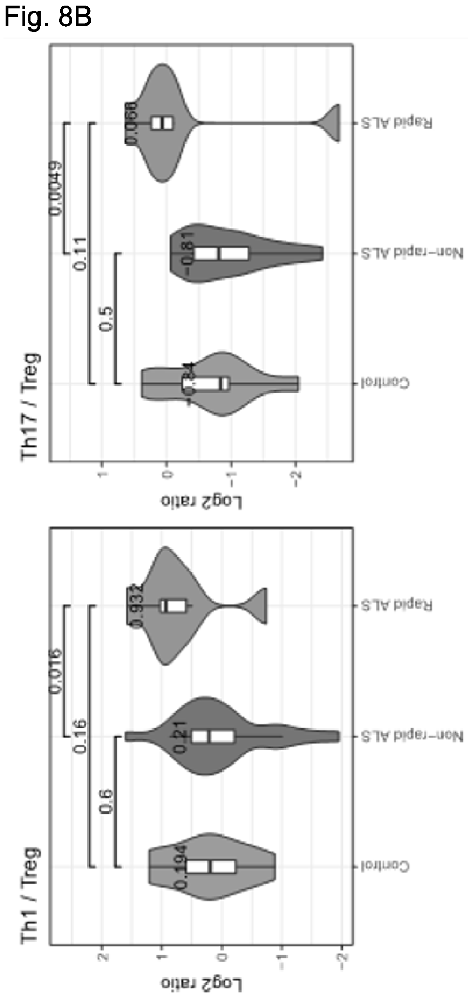

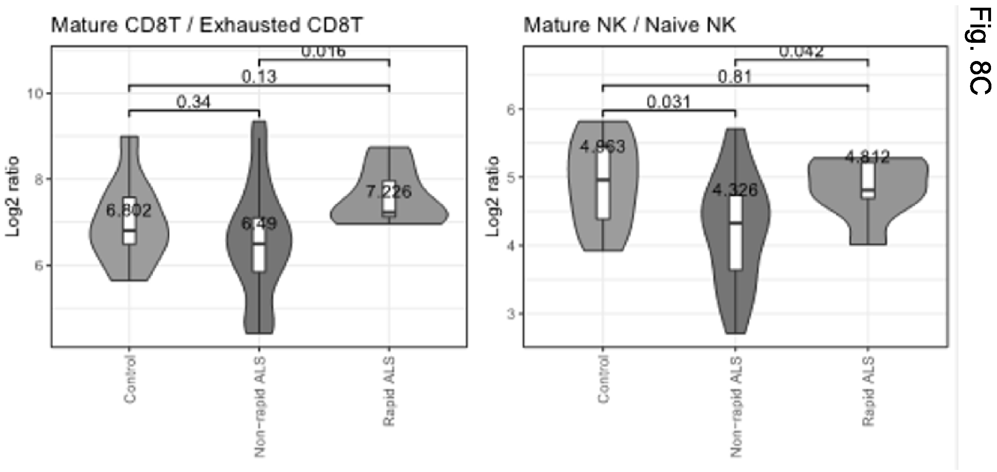

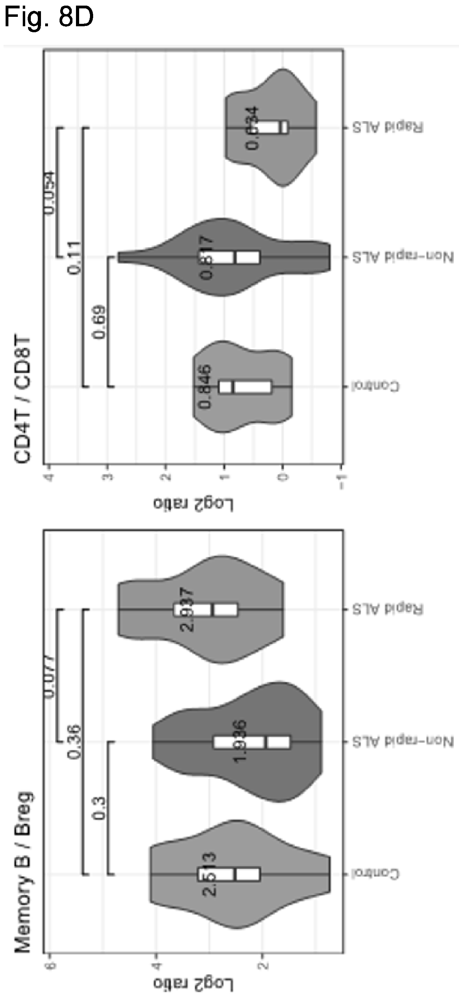

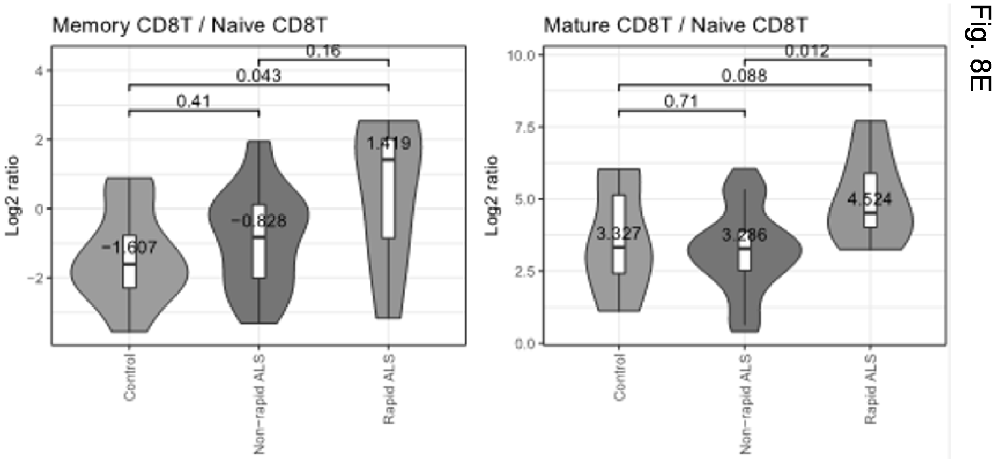

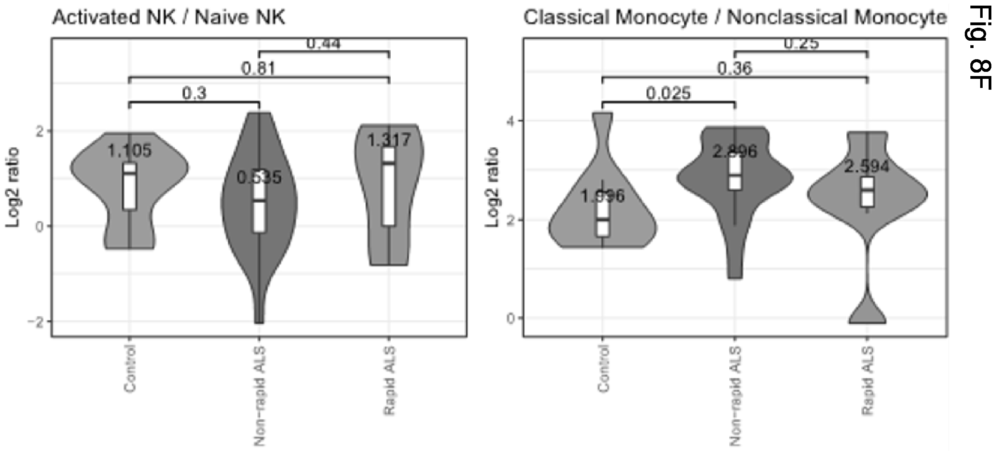

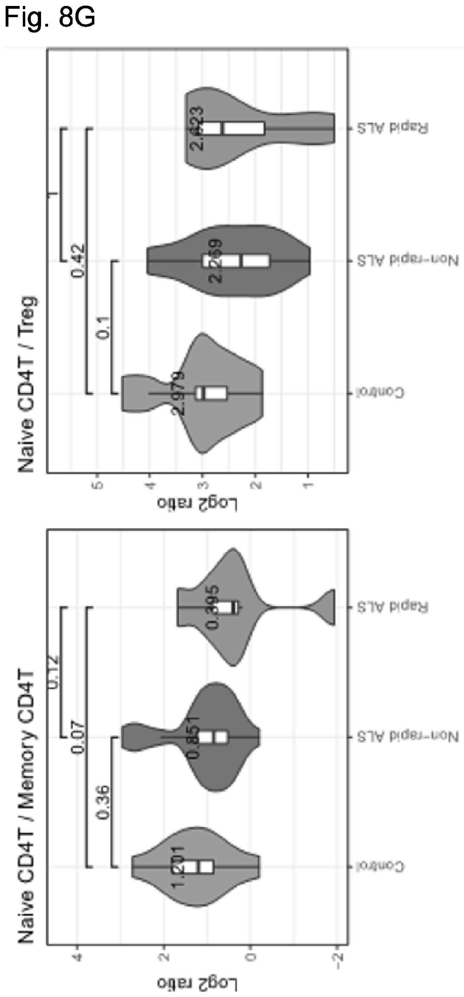

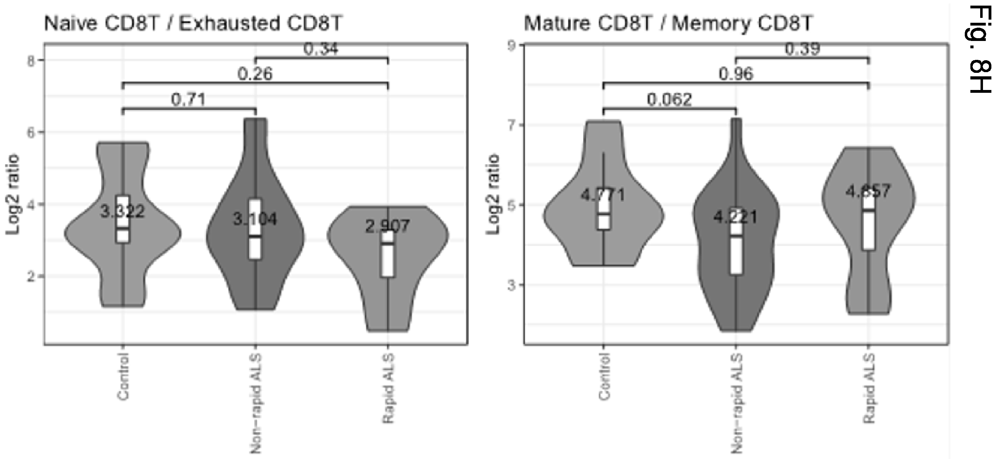

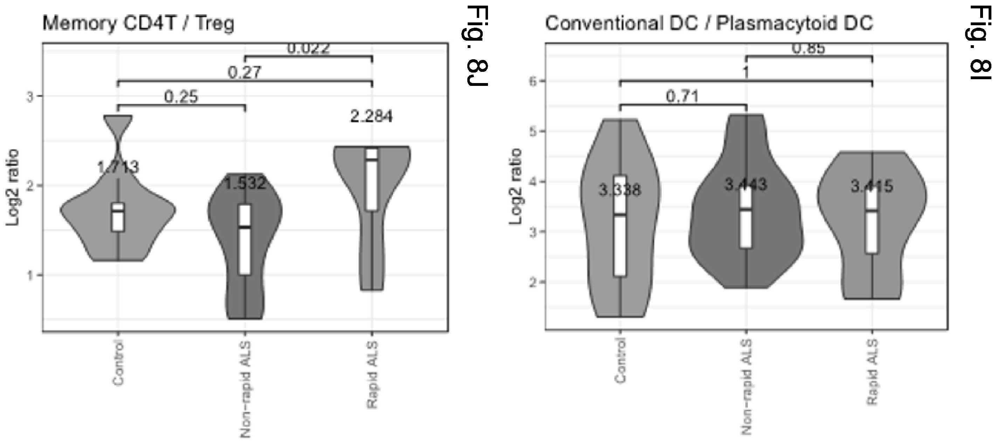

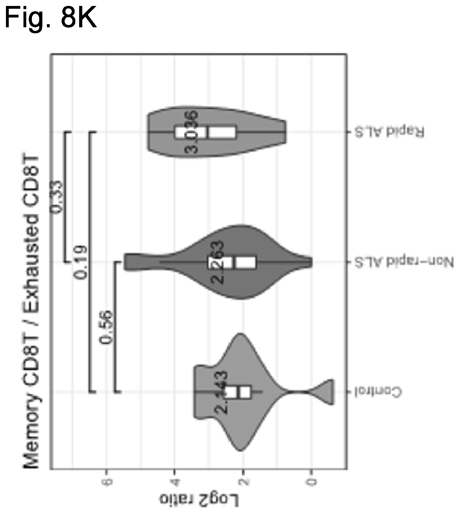

Integrated scRNA-seq data were classified into 23 cell types (Figure 5) and selected for further analysis (Figure 1B). T helper 2 cells (Th2) were not separated as a cluster. To examine immune profiles, first, the frequency of cell types in all cells of controls, non-rapid ALS, and rapid ALS was calculated (Figure 1C and Figure 6). The number of Treg was significantly increased in non-rapid ALS compared to controls and rapid ALS (2.4% vs. 1.9%, p=0.011, and 2.4% vs. 1.6%, p=0.029, respectively; Figure 2A). Next, the frequency of cell types of related cells in all CD4 T cells, such as naive CD4 T cells, memory CD4 T cells, Th1, Th17, or Treg, was calculated (Figure 7). The frequency of mature CD8 T cells in all CD8 T cells was significantly higher in rapid ALS than in non-rapid ALS (91.6% vs. 82.2%, p=0.042). On the other hand, the frequency of regulatory B cells in all B cells was significantly lower in rapid ALS than in non-rapid ALS (5.5% vs. 7.3%, p=0.048), and the frequency of naive CD8 T cells and exhausted CD8 T cells in all CD8 T cells was significantly lower in rapid ALS than in non-rapid ALS (4.1% vs. 8.4%, p=0.010, and 0.6% vs. 0.9%, p=0.042, respectively) (Figure 2B). On the other hand, in all CD4 T cells, there was no significant difference in the frequency of Treg between rapid ALS and non-rapid ALS (6.8% vs. 8.5%, p=0.31; Figure 7). Furthermore, frequency ratios between functionally related immune cells were calculated (Figure 8). The ratios of memory CD4 T cells/Treg (4.9 vs. 2.9, p=0.022), Th1/Treg (1.9 vs. 1.2, p=0.016), Th17/Treg (1 vs. 0.6, p=0.0049), mature CD8 T cells/naive CD8 T cells (23.0 vs. 9.8, p=0.012), mature CD8 T cells/exhausted CD8 T cells (149.7 vs. 89.9, p=0.016), and mature natural killer cells/naive natural killer cells (28.1 vs. 20.1, p=0.042) were significantly higher in rapid ALS than in non-rapid ALS (Figure 2C). These results showed that the immunological profiles of rapid ALS and non-rapid ALS are different.

### Immune Cell Frequency Correlated with Progression Rate

Next, the relationship between the frequency of each cell type and ΔALSFRS-R/month was examined for 30 ALS patients. There was a significant correlation between ΔALSFRS-R/month and age (Pearson's correlation coefficient = 0.421, p = 0.021), and since confounding with age cannot be ignored in routine correlation analysis, partial correlation was used. Treg in all cells tended to show a negative correlation with progression rate, but was not significant (coefficient = -0.351, p = 0.062) (Table 4). On the other hand, the frequency of Th17 in CD4 T cells significantly correlated with ΔALSFRS-R/month (coefficient = 0.3898, p = 0.0366; Table 5). Furthermore, the ratios of memory CD4 T cells/Treg (coefficient = 0.450, p = 0.014) and Th17/Treg (coefficient = 0.428, p = 0.021) significantly correlated with ΔALSFRS-R/month (Table 6).

### Table 4. Partial Correlation Analysis between Frequency of Each Cell Type in All Cells and ΔALSFRS-R/month

**[Table 4]**

| **Cell Type** | **Partial Correlation Coefficient** | **P-value** |
|---|---|---|
| Treg | -0.351 | 0.062 |
| Classical DC | -0.249 | 0.193 |
| Th17 | 0.246 | 0.198 |
| Mature CD8 T | 0.235 | 0.220 |
| Naive NK | -0.188 | 0.329 |
| Naive B | -0.177 | 0.358 |
| Naive CD4 T | -0.176 | 0.362 |
| Exhausted CD8 T | -0.166 | 0.390 |
| Plasmacytoid DC | -0.163 | 0.400 |
| Breg | -0.157 | 0.416 |
| Th1 | 0.145 | 0.453 |
| Memory CD8 T | 0.144 | 0.456 |
| Macrophage | -0.126 | 0.516 |
| γ**Δ**T | -0.118 | 0.541 |
| Classical monocyte | 0.115 | 0.553 |
| Memory CD4 T | 0.100 | 0.606 |
| Mature NK | -0.084 | 0.667 |
| Memory B | -0.073 | 0.707 |
| Non-classical monocyte | 0.056 | 0.771 |
| Activated NK | -0.053 | 0.787 |
| Plasma | 0.046 | 0.814 |
| Naive CD8 T | -0.018 | 0.925 |
| Platelet | 0.011 | 0.956 |

| | | |
|---|---|---|
| DC = dendritic cell, NK = natural killer. | | |

### Table 5. Partial Correlation Analysis between Cell Type Frequency in Each Immune Cell and ΔALSFRS-R/month

**[Table 5]**

| **Population** | **Cell Type** | **Partial Correlation Coefficient** | **P-value** |
|---|---|---|---|
| CD4 T | Th17 | 0.3898 | 0.0366 |
| CD4 T | Memory CD4 T | 0.3338 | 0.0768 |
| T | CD8 T | 0.3061 | 0.1063 |
| T | CD4 T | -0.3051 | 0.1075 |
| CD4 T | Naive CD4 T | -0.2689 | 0.1584 |
| CD8 T | Exhausted CD8 T | -0.2459 | 0.1984 |
| B | Breg | -0.2291 | 0.2319 |
| CD4 T | Th1 | 0.2169 | 0.2585 |
| CD4 T | Treg | -0.2012 | 0.2952 |
| B | Memory B | 0.1948 | 0.3112 |
| NK | Naive NK | -0.1636 | 0.3966 |
| B | Naive B | -0.1565 | 0.4177 |
| T | γ**Δ**T | -0.1080 | 0.5772 |
| CD8 T | Mature CD8 T | 0.1079 | 0.5775 |
| NK | Activated NK | 0.0971 | 0.6165 |
| CD8 T | Naive CD8 T | -0.0921 | 0.6348 |
| Monocyte | Classical monocyte | 0.0694 | 0.7207 |
| Monocyte | Non-classical monocyte | -0.0694 | 0.7207 |
| NK | Mature NK | 0.0326 | 0.8666 |
| DC | Classical DC | -0.0058 | 0.9760 |
| DC | Plasmacytoid DC | 0.0058 | 0.9760 |
| CD8 T | Memory CD8 T | -0.0002 | 0.9991 |

| | | | |
|---|---|---|---|
| DC = dendritic cell, NK = natural killer. | | | |

### Table 6. Partial Correlation Analysis between Frequency Ratio and ΔALSFRS-R/month

**[Table 6]**

| **Cell Type** | **Partial Correlation Coefficient** | **P-value** |
|---|---|---|
| Memory CD4 T / Treg | 0.450 | 0.014 |
| Th17 / Treg | 0.428 | 0.021 |
| Th1 / Treg | 0.350 | 0.063 |
| Naive CD4 T / Memory CD4 T | -0.315 | 0.096 |
| CD4 T / CD8 T | -0.307 | 0.105 |
| Mature CD8 T / Exhausted CD8 T | 0.278 | 0.144 |
| Naive CD8 T / Mature CD8 T | -0.242 | 0.206 |
| Memory B / Breg | 0.233 | 0.224 |
| Naive NK / Activated NK | -0.185 | 0.336 |
| Naive NK / Mature NK | -0.180 | 0.350 |
| Exhausted CD8 T / Memory CD8 T | -0.153 | 0.428 |
| Naive B / Memory B | -0.150 | 0.438 |
| Mature CD8 T / Memory CD8 T | 0.138 | 0.474 |
| Naive CD8 T / Memory CD8 T | -0.111 | 0.568 |
| Naive B / Breg | 0.087 | 0.653 |
| Classical monocyte / Non-classical monocyte | 0.054 | 0.780 |
| Naive CD4 T / Treg | -0.026 | 0.894 |
| Classical DC / Plasmacytoid DC | 0.025 | 0.898 |
| Naive CD8 T / Exhausted CD8 T | 0.005 | 0.981 |

| | | |
|---|---|---|
| DC = dendritic cell, NK = natural killer. | | |

### Few Differentially Expressed Genes Were Found in Immune Cells of ALS

The association between ALS and qualitative changes in immune cells was also examined. To understand the overall qualitative changes in each group, DEG analysis was performed using scRNA-seq data. Differential expression analysis of scRNA-seq data was performed between ALS vs. controls, non-rapid ALS vs. controls, rapid ALS vs. controls, and rapid ALS vs. non-rapid ALS, and then DEGs were isolated in 23 cell types (Bonferroni adjusted p < 0.05, fold change ≥ 1.2). However, there were few DEGs identified in each comparison, and none were known to be immune-related genes (Table 7).

### Table 7. List of Numbers of Differentially Expressed Gene

**[Table 7]**

| **Cell Type** | **ALS vs. Controls** | **Non-rapid ALS vs. Controls** | **Rapid ALS vs. Controls** | **Rapid ALS vs. Non-rapid ALS** |
|---|---|---|---|---|
| Naive B | 0 | 0 | 0 | 0 |
| Memory B | 0 | 0 | 1 | 0 |
| Breg | 0 | 0 | 0 | 0 |
| Plasma | 0 | 0 | 0 | 0 |
| Naive CD4 T | 1 | 1 | 1 | 0 |
| Memory CD4 T | 0 | 0 | 0 | 0 |
| Th1 | 0 | 0 | 1 | 0 |
| Th17 | 0 | 0 | 0 | 0 |
| Treg | 0 | 0 | 0 | 0 |
| Naive CD8 T | 0 | 0 | 0 | 0 |
| Mature CD8 T | 0 | 0 | 0 | 0 |
| Memory CD8 T | 0 | 0 | 0 | 0 |
| Exhausted CD8 T | 0 | 0 | 0 | 0 |
| γ**Δ**T | 0 | 0 | 1 | 0 |
| Naive NK | 0 | 0 | 0 | 0 |
| Mature NK | 0 | 0 | 0 | 0 |
| Activated NK | 0 | 0 | 0 | 0 |
| Classical monocyte | 1 | 1 | 1 | 0 |
| Non-classical monocyte | 0 | 0 | 0 | 0 |
| Macrophage | 1 | 1 | 1 | 0 |
| Classical DC | 0 | 0 | 0 | 0 |
| Plasmacytoid DC | 0 | 0 | 1 | 0 |
| Platelet | 9 | 6 | 10 | 0 |

| | | | | |
|---|---|---|---|---|
| ALS = amyotrophic lateral sclerosis, DC = dendritic cell, NK = natural killer. | | | | |

### Immune-related Proteins Correlated with Progression Rate

Killer cell lectin-like receptor D1 (KLRD1; fold change = 2.2, p < 0.001), trefoil factor 2 (TFF2; fold change = 2.3, p < 0.001), keratin 19 (KRT19; fold change = 3.2, p < 0.001), interleukin-17A (IL-17A; fold change = 4.8, p = 0.007), YTH N6-methyladenosine RNA binding protein F3 (YTHDF3; fold change = 2.6, p = 0.025), and neutrophil cytosolic factor 2 (NCF2; fold change = 2.4, p = 0.041) were identified to be significantly elevated in rapid vs. non-rapid ALS. Of these, KLRD1 (fold change = 2.2, p < 0.001), KRT19 (fold change = 2.7, p = 0.01), NCF2 (fold change = 3.0, p = 0.018), YTHDF3 (fold change = 3.0, p = 0.017), and IL-17A (fold change = 4.0, p = 0.037) were also significantly elevated in rapid ALS vs. controls (Figure 3A, B). The expression level of pNf-H was significantly higher in ALS (non-rapid + rapid ALS) vs. controls (fold change = 180, p = 0.001) and in rapid ALS vs. controls (fold change = 3074, p < 0.001) (Figure 3A, C), which supported the validity of our samples. On the other hand, pNf-H levels showed no difference between rapid ALS and non-rapid ALS, suggesting that pNf-H is non-specific to progression rate in ALS.

Furthermore, partial correlation analysis between serum protein levels and ΔALSFRS-R/month was performed to examine whether the measured proteins correlate with disease progression. 82 proteins had significant partial correlations with ΔALSFRS-R/month (Table 8). All six DEPs that were significantly elevated in rapid ALS vs. non-rapid ALS or controls had significant partial correlations, and especially KLRD1, KRT19, and IL-17A had coefficients of 0.5 or more and p-values of 0.003 or less. IL-17C and IL-12 receptor subunit β1 showed less partial correlation (coefficient = 0.416 and p = 0.031, coefficient = 0.383 and p = 0.049, respectively). On the other hand, IL-17D, IL-17F, and IL-17 receptor B did not show significant correlation.

### Table 8. Partial Correlation Analysis between Serum Proteome and ΔALSFRS-R/month

**[Table 8-1]**

| **Protein** | **Partial Correlation Coefficient** | **P-value** | **Protein** | **Partial Correlation Coefficient** | **P-value** | **Protein** | **Partial Correlation Coefficient** | **P-value** |
|---|---|---|---|---|---|---|---|---|
| KLRD1 | 0.745 | 0.000 | B4GALT1 | 0.483 | 0.008 | WNT9A | 0.428 | 0.026 |
| CEP164 | 0.697 | 0.000 | PLAUR | 0.483 | 0.008 | PTPRM | 0.424 | 0.027 |
| CD160 | 0.665 | 0.000 | IL2 | 0.498 | 0.008 | LILRB4 | 0.424 | 0.027 |
| KRT19 | 0.658 | 0.000 | NCF2 | 0.498 | 0.008 | CCL25 | 0.412 | 0.029 |
| TNFRSF11A | 0.644 | 0.000 | IFNGR1 | 0.478 | 0.009 | TNFRSF11B | 0.402 | 0.031 |
| CD79B | 0.610 | 0.001 | TNFRSF13B | 0.477 | 0.009 | IL17C | 0.416 | 0.031 |
| CLEC7A | 0.594 | 0.001 | YTHDF3 | 0.492 | 0.009 | GOPC | 0.410 | 0.033 |
| TFF2 | 0.584 | 0.001 | HCLS1 | 0.481 | 0.010 | CD83 | 0.410 | 0.034 |
| CKAP4 | 0.585 | 0.001 | PPP1R9B | 0.475 | 0.011 | IL18R1 | 0.399 | 0.035 |
| CSF1 | 0.554 | 0.002 | ADGRE2 | 0.467 | 0.011 | NRTN | 0.406 | 0.036 |
| F2R | 0.550 | 0.002 | LTBR | 0.467 | 0.011 | LY6D | 0.391 | 0.036 |
| BTN3A2 | 0.563 | 0.002 | LSP1 | 0.481 | 0.011 | RAB37 | 0.401 | 0.038 |
| IL17A | 0.556 | 0.003 | FOXO1 | 0.478 | 0.012 | CRELD2 | 0.386 | 0.038 |
| LAIR1 | 0.536 | 0.003 | SAMD9L | 0.477 | 0.012 | IL4R | 0.400 | 0.039 |
| IKBKG | 0.548 | 0.003 | TNFRSF14 | 0.459 | 0.012 | SIGLEC10 | 0.399 | 0.039 |
| CD4 | 0.541 | 0.004 | SRPK2 | 0.475 | 0.012 | JUN | 0.393 | 0.042 |
| NPPC | 0.540 | 0.004 | ITM2A | 0.467 | 0.014 | MZB1 | 0.378 | 0.043 |

**[Table 8-2]**

| | | | | | | | | |
|---|---|---|---|---|---|---|---|---|
| COL9A1 | 0.538 | 0.004 | COLEC12 | 0.443 | 0.016 | ITGA11 | -0.390 | 0.045 |
| FCRL6 | 0.533 | 0.004 | CD40 | 0.437 | 0.018 | IDS | -0.381 | 0.045 |
| CEACAM21 | 0.531 | 0.004 | LIFR | 0.453 | 0.018 | CTSO | 0.379 | 0.047 |
| CCL23 | 0.512 | 0.004 | PTPN6 | 0.435 | 0.018 | TNFSF10 | 0.378 | 0.047 |
| SERPINB8 | 0.518 | 0.005 | TNFSF13 | 0.431 | 0.019 | SCGN | 0.384 | 0.048 |
| FSTL3 | 0.504 | 0.005 | CHRDL1 | 0.429 | 0.020 | IL12RB1 | 0.383 | 0.049 |
| BACH1 | 0.518 | 0.006 | EGLN1 | 0.442 | 0.021 | OSCAR | 0.368 | 0.049 |
| GAL | -0.492 | 0.007 | TNFB | -0.475 | 0.022 | | | |
| ESM1 | 0.491 | 0.007 | LGALS9 | 0.424 | 0.022 | | | |
| FLT3LG | -0.498 | 0.007 | IL10RB | 0.420 | 0.023 | | | |
| AGRN | 0.490 | 0.007 | PSIP1 | 0.432 | 0.025 | | | |
| KLRB1 | 0.497 | 0.007 | LGALS4 | 0.415 | 0.025 | | | |

### Results of scRNA-seq and Serum Immunoproteomics Were Correlated

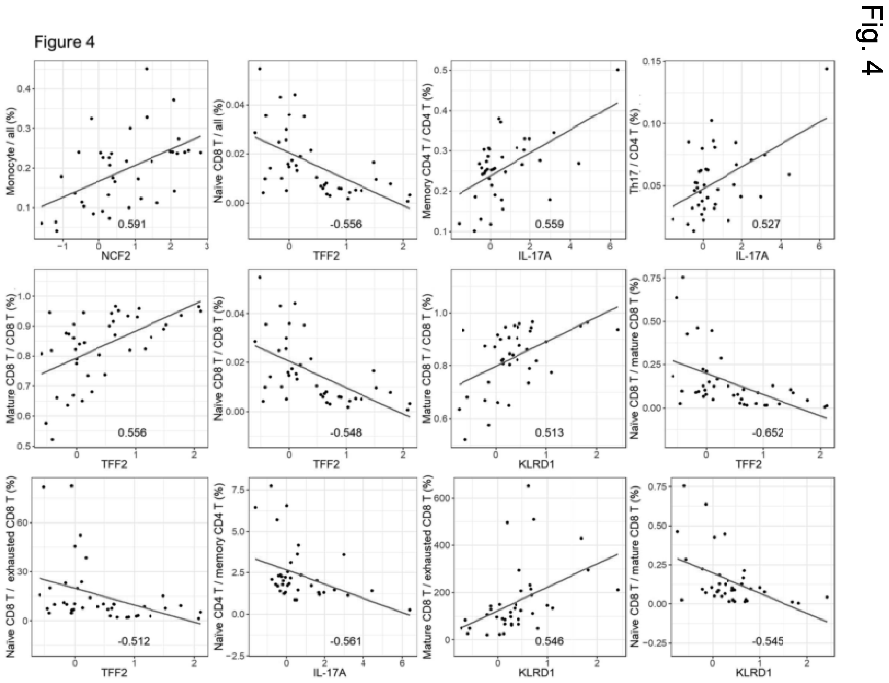

As an integrated analysis of scRNA-seq and immunoproteomics, correlation analysis was performed between the expression levels of serum proteins and the frequency/ratio of each cell type analyzed in scRNA-seq analysis (Table 9). Combinations of DEPs and cell types that were significantly increased in rapid ALS compared to non-rapid ALS or controls with a correlation coefficient > 0.5 were NCF2-classical monocyte, TFF2-mature CD8 T cells, KLRD1-mature CD8 T cells, IL-17A-memory CD4 T cells, and IL-17A-Th17 (Figure 4).

### Table 9. Correlation between Serum Proteome and Cell Type in scRNA-seq

**[Table 9-1]**

| In all cells | | | | In cell subsets | | |
|---|---|---|---|---|---|---|
| Protein | Cell Type | Correlation | | Protein | Cell Type | Correlation |
| HGF | Classical monocyte | 0.756 | | BTN3A2 | Memory CD4T | 0.620 |
| OSM | Naive CD4T | -0.660 | | IL2RB | Th17 | 0.601 |
| OSM | Classical monocyte | 0.641 | | PCDH1 | Treg | -0.594 |
| TGFalpha | Classical monocyte | 0.628 | | OSM | Naive CD4T | -0.578 |
| TGFA | Classical monocyte | 0.606 | | KYNU | Treg | 0.570 |
| PRKAB1 | Naive NK | 0.597 | | CLEC4C | gdT | 0.567 |
| HGF | Naive CD4T | -0.592 | | BTN3A2 | Naive CD4T | -0.563 |
| NCF2 | Classical monocyte | 0.591 | | IL17A | Memory CD4T | 0.559 |
| IL1RN | Naive CD4T | -0.586 | | PADI2 | Mature NK | -0.559 |
| IL1RN | Classical monocyte | 0.580 | | FGF21 | Treg | 0.557 |
| TPSAB1 | Classical DC | -0.578 | | TFF2 | Mature CD8T | 0.556 |

**[Table 9-2]**

| In all cells | | | | In cell subsets | | |
|---|---|---|---|---|---|---|
| TNFRSF11B | Naive CD8T | -0.574 | | TNFRSF11B | Mature CD8T | 0.556 |
| TGFA | Naive CD4T | -0.573 | | PLAUR | Memory CD4T | 0.555 |
| CLEC4D | Classical monocyte | 0.563 | | CSF3 | Th17 | 0.551 |
| TFF2 | Naive CD8T | -0.556 | | TFF2 | Naive CD8T | -0.548 |
| IFNLR1 | Th17 | -0.556 | | TNFRSF14 | Memory CD4T | 0.547 |
| TNFRSF11B | Breg | -0.550 | | SPRY2 | gdT | 0.545 |
| IFNLR1 | Memory CD4T | -0.549 | | LILRB4 | Memory CD4T | 0.542 |
| CASP8 | Macrophage | 0.548 | | FSTL3 | Memory CD4T | 0.537 |
| EGLN1 | Classical monocyte | 0.542 | | F2R | Memory CD4T | 0.536 |
| Flt3L | Mature CD8T | -0.538 | | TGFA | gdT | 0.536 |
| SRPK2 | Classical monocyte | 0.532 | | IL18 | gdT | 0.535 |
| TGFalpha | Naive CD4T | -0.531 | | GZMB | Treg | 0.532 |
| TNFRSF11B | Naive B | -0.530 | | OSM | Memory CD4T | 0.530 |
| ROBO1 | Memory CD4T | -0.529 | | PLAUR | Naive CD4T | -0.528 |
| CCL25 | Naive CD8T | -0.528 | | HGF | Classical monocyte | 0.528 |

**[Table 9-3]**

| In all cells | | | | In cell subsets | | |
|---|---|---|---|---|---|---|
| TNFSF14 | Classical monocyte | 0.524 | | HGF | Non-classical monocyte | -0.528 |
| LTA | gdT | 0.524 | | IL17A | Th17 | 0.527 |
| CCN2 | Platelet | 0.524 | | TNFRSF9 | gdT | 0.527 |
| BACH1 | Classical monocyte | 0.516 | | HGF | Naive CD4T | -0.526 |
| TREM2 | Naive CD8T | -0.515 | | IL18 | Treg | 0.525 |
| NBN | Classical monocyte | 0.513 | | CEACAM21 | Memory CD4T | 0.517 |
| IL1R2 | Memory CD4T | -0.511 | | CDCP1 | Memory CD8T | -0.517 |
| LAT | Breg | 0.511 | | CDCP1 | Mature CD8T | 0.515 |
| PLAUR | Classical monocyte | 0.509 | | CRIM1 | Memory CD4T | 0.514 |
| HCLS1 | Classical monocyte | 0.507 | | TLR3 | Activated NK | -0.514 |
| LSP1 | Classical monocyte | 0.505 | | KLRD1 | Mature CD8T | 0.513 |
| LSP1 | Naive CD4T | -0.503 | | IFNgamma | Classical monocyte | -0.511 |
| SPRY2 | Mature CD8T | -0.501 | | IFNgamma | Non-classical monocyte | 0.511 |
| FGF5 | Naive CD8T | -0.501 | | LRRN1 | Mature CD8T | -0.510 |
| | | | | SULT2A1 | Treg | 0.509 |
| | | | | IL1RN | Treg | 0.508 |
| | | | | IL4R | Memory CD4T | 0.504 |

**[Table 9-4]**

| In ratios between cell subsets | | |
|---|---|---|
| Protein | Cell Type | Correlation |
| TFF2 | Naive CD8T / Mature CD8T | -0.652 |
| BTN3A2 | Naive CD4T / Memory CD4T | -0.612 |
| TNFRSF11B | Naive CD8T / Mature CD8T | -0.606 |
| SPINT2 | Naive CD8T / Exhausted CD8T | -0.603 |
| TNFRSF11B | Naive CD8T / Exhausted CD8T | -0.585 |
| PCDH1 | Memory CD4T / Treg | 0.584 |
| PCDH1 | Th17 / Treg | 0.578 |
| ESM1 | Naive CD8T / Mature CD8T | -0.573 |
| IL1RN | Naive CD4T / Treg | -0.566 |
| OSM | Naive CD4T / Treg | -0.566 |
| MLN | Naive CD8T / Exhausted CD8T | -0.563 |
| IL17A | Naive CD4T / Memory CD4T | -0.561 |
| HGF | Naive CD4T / Treg | -0.559 |
| OSM | Naive CD4T / Memory CD4T | -0.558 |
| PLAUR | Naive CD4T / Memory CD4T | -0.556 |
| BTN3A2 | Naive CD8T / Exhausted CD8T | -0.554 |
| CDCP1 | Naive CD4T / Treg | -0.552 |
| IFNGR1 | Memory CD4T / Treg | 0.547 |

**[Table 9-5]**

| In ratios between cell subsets | | |
|---|---|---|
| KLRD1 | Mature CD8T / Exhausted CD8T | 0.546 |
| KLRD1 | Naive CD8T / Mature CD8T | -0.545 |
| KYNU | Memory CD4T / Treg | -0.540 |
| GZMB | Th1 / Treg | -0.540 |
| TNFRSF9 | Naive CD8T / Exhausted CD8T | -0.536 |
| CD200 | Memory CD4T / Treg | 0.532 |
| F2R | Naive CD4T / Memory CD4T | -0.530 |
| DECR1 | Naive NK / Activated NK | 0.527 |
| CSF1 | Mature CD8T / Memory CD8T | 0.525 |
| CCL25 | Naive CD8T / Mature CD8T | -0.524 |
| TNFRSF14 | Naive CD4T / Memory CD4T | -0.523 |
| CRIM1 | Naive CD8T / Mature CD8T | -0.522 |
| HGF | Naive CD4T / Memory CD4T | -0.520 |
| LIFR | Naive B / Breg | 0.518 |
| TREM2 | Naive CD4T / Treg | -0.516 |
| TFF2 | Naive CD8T / Exhausted CD8T | -0.514 |
| MILR1 | Naive CD8T / Mature CD8T | -0.512 |
| HGF | Classical monocyte / Non-classical monocyte | 0.512 |
| CSF1 | Naive CD8T / Mature CD8T | -0.512 |
| CEACAM21 | Naive CD8T / Mature CD8T | -0.511 |
| F2R | Naive CD8T / Mature CD8T | -0.511 |
| PCDH1 | Th1 / Treg | 0.509 |
| BTN3A2 | Naive CD4T / Treg | -0.506 |
| KYNU | Th1 / Treg | -0.505 |
| BTN3A2 | Naive CD8T / Mature CD8T | -0.503 |

### Discussion

In this study, multi-omics was applied to peripheral immune profiling to determine how immunological changes in ALS patients are related to disease progression rate. By scRNA-seq, cell type shifts to Th17 vs. Treg, mature CD8 T cells vs. naive CD8 T cells, and mature natural killer cells vs. naive natural killer cells were revealed in rapidly progressive ALS. By serum immunoproteomics, Th17-related proteins and CD8 T cell-related proteins were significantly elevated in rapidly progressive ALS and correlated with disease progression rate. Finally, in integrated analysis of scRNA-seq and immunoproteomics, dynamic associations were shown between specific immune cells and proteins, such as Th17 and IL-17A, and mature CD8 T cells and KLRD1.

Th17 can promote immunity against many pathogens, but can also promote inflammatory pathology in infections and autoimmunity. From the few studies of flow cytometric analysis using PBMC, it has been shown that ALS patients have higher frequencies of Th1 and Th17 and lower frequencies of Th2 and Treg compared to healthy controls. However, previous studies have not clarified the relationship between Th17 and ALS progression rate. This time, we showed that an increase in Th17 in CD4 T cells is associated with rapid progression of ALS. We also revealed a significant correlation between the ratio of Th17 to Treg and progression rate. On the other hand, Treg frequency alone did not show a significant correlation with progression rate. Therefore, a shift to Th17 compared to Treg, rather than a simple decrease in Treg, would be important for rapid progression of ALS.

Treg frequency was significantly decreased in rapid ALS vs. non-rapid ALS, but there was no difference between rapid ALS and controls. Also, as mentioned above, Treg frequency did not show a significant correlation with progression rate. These results do not support the idea that Treg reduction is mainly related to rapid progression. Clinical trial results using Treg in ALS have been reported. In rapid progression, IL-17C and IL-17F were elevated, and progression and cytokine levels were not suppressed by Treg administration. This suggests that Treg has little effect on rapid progression associated with elevated IL-17 cytokine family.

Proteomic analysis revealed that KLRD1, TFF2, KRT19, IL-17A, YTHDF3, and NCF2 were elevated in rapid ALS vs. non-rapid ALS. Most of these also correlated with the frequency of specific cell types analyzed by scRNA-seq. First, IL-17A values correlated with memory CD4 T cell and Th17 frequencies, suggesting that Th17 actually affects disease progression through its major effector IL-17A. Although increased Th17 or elevated IL-17A in serum or CSF has been reported, the association of rapid progression with these has remained unclear. In this study, comprehensive analysis using scRNA-seq and immunoproteomics succeeded in associating Th17 and IL-17A (but not IL-17F) with rapid progression of ALS. More notably, it has been reported that IL-17A signaling pathway is associated with regulation of KRT19 in breast cancer development.

Second, KLRD1 value showed the highest partial correlation coefficient with progression rate among 400 immune proteins, and correlated with mature CD8 T cell frequency. This result is consistent with increased mature CD8 T cell frequency in rapid ALS. Similarly, KLRD1 has been reported as one of the genes differentially expressed in activated CD8 T cells in CSF of ALS patients vs. controls. TFF2 value also correlates with mature CD8 T cell frequency. Previous studies have shown that CD8 T cells are increased in ALS patients, and higher proportions of CD8 T cells are associated with higher ALS mortality risk, but the relationship between CD8 T cells and ALS progression has been unclear. In view of this situation, it is noteworthy that clonally expanded, terminally differentiated effector memory (T_{EMRA}) CD8 T cells of autoreactive origin in blood and CNS are associated with ALS4 in mice and humans caused by mutations in the senataxin gene (SETX). Taken together, specific types of CD8 T cells may be involved in ALS progression or pathophysiology, and further research is needed.

Third, NCF2 value correlated with classical monocyte frequency. NCF2 is involved in superoxide synthesis in neutrophils and has been reported as one of the ferroptosis and iron metabolism-related genes differentially expressed in ALS and controls. Regarding monocytes, although no association between monocytes and progression rate has been shown, it has been reported that the ratio of classical monocytes to non-classical monocytes is elevated in ALS patients.

Some limitations of this study are noted. First, patients were called from various regions in Japan, but participants were recruited at a single center. The number of participants was relatively small, but comparable to other recent studies of scRNA-seq. Second, patients without a family history of ALS at enrollment were classified as sporadic, and genetic testing was not necessarily performed. As a result, two patients were found to have SOD1-ALS and were excluded from analysis because they were from the same family. On the other hand, the possibility of C9orf72-ALS was extremely low in Japanese cases. Third, disease duration was shorter and ALSFRS-R scores at enrollment were lower in rapid ALS than in non-rapid ALS. Although the association of rapid progression in a short period with more severe symptoms was reasonable, these could be confounding factors. Fourth, in relation to the above issues, long-term evaluation was beyond the scope of this study. Additional evaluation at different stages would clarify temporal, progressive changes in immune profiles in ALS.

In summary, multi-omics of scRNA-seq and PEA-based immunoproteomics demonstrated a clear association between rapid progression in ALS patients and increased Th17 vs. Treg, mature CD8 T cells vs. naive CD8 T cells, IL-17A-related proteins, and CD8 T cell-related proteins in peripheral blood. Also shown was the correspondence between specific cell types and associated immune proteins. These results can provide promising targets and biomarkers for disease-modifying therapy of ALS.

### INDUSTRIAL APPLICABILITY

According to the present invention, biomarkers for use in determining or diagnosing the progression rate or likelihood of amyotrophic lateral sclerosis (ALS), and/or in selecting or predicting therapeutic agents can be provided. The present invention can be utilized in the medical field and the like.

## Claims

1. A biomarker for use in determining or diagnosing the progression rate or likelihood of amyotrophic lateral sclerosis (ALS), and/or in selecting or predicting a therapeutic agent, wherein the biomarker is selected from the group consisting of IL-17A, KLRD1, KRT19, NCF2, TFF2, YTHDF3, Th17, regulatory T cells (Treg), mature CD8 T, naive CD8 T, exhausted CD8 T, classical monocyte, memory CD4 T, Th17/Treg, mature CD8 T/naive CD8 T, and mature CD8 T/exhausted CD8 T.

2. The biomarker according to claim 1, for use in determining or diagnosing rapidly progressive amyotrophic lateral sclerosis (ALS).

3. The biomarker according to claim 1, wherein the biomarker is selected from combinations consisting of IL-17A and Th17, IL-17A and memory CD4 T, KLRD1 and mature CD8 T, TFF2 and mature CD8 T, and NCF2 and classical monocyte.

4. A method for determining or diagnosing the progression rate or likelihood of amyotrophic lateral sclerosis (ALS) in a subject, comprising determining the level of the biomarker according to any one of claims 1 to 3 in a sample derived from said subject.

5. The method according to claim 4, further comprising determining or diagnosing the progression rate or likelihood of ALS based on a reference level of said biomarker.

6. The method according to claim 5, wherein the reference level is the level of said biomarker in a sample derived from a subject without ALS, a sample derived from a subject with ALS, a sample derived from a subject with non-rapidly progressive ALS, or a sample derived from a subject with rapidly progressive ALS.
